# EUROPEAN PATENT APPLICATION

(11) **EP 4 803 012 A1**
(43) Date of publication of application: **09.09.2026**
(21) Application number: 25161189.3
(22) Date of filing: 03.03.2025
(51) Int. Cl.: A61B 8/08, A61B 8/12, A61B 8/14, A61B 8/00

(54) **RECTANGULAR/ELONGATED ARRAY IN ULTRASOUND IMAGING WITH USER GUIDANCE FOR HIGHER RESOLUTION ANATOMICAL VIEW PLANES**

(71) Applicant: Koninklijke Philips N.V., 5656 AE Eindhoven (NL)
(72) Inventor: WEBER, Frank Michael, Eindhoven (NL); BROWN, Lynne Mary, Eindhoven (NL)
(74) Representative: Philips Intellectual Property & Standards

(57) **Abstract**

The present disclosure may include devices, systems, apparatuses, and methods for capturing improved 2D images of desired view planes. For example, an apparatus is disclosed that includes a rectangular ultrasound array and a processor configured for communication therewith. The processor may be configured to: control the rectangular ultrasound array to obtain 3D ultrasound data of an anatomy of a patient while the rectangular ultrasound array is in a first pose; determine a location of a 2D view plane of the anatomy within a 3D model of the anatomy based on at least the 3D ultrasound data; determine, for the 2D view plane, a first scan angle associated with the first pose of the rectangular ultrasound array; determine, for the first scan angle, a first image quality; and output a screen display comprising the location of the 2D view plane, the first scan angle, and/or the first imaging quality.

## Description

### TECHNICAL FIELD

The present disclosure relates generally to devices, systems, and methods of performing ultrasonic imaging and, in particular, performing ultrasonic imaging with a rectangular or otherwise elongated array of ultrasound transducer elements.

### BACKGROUND

Diagnostic and therapeutic ultrasound catheters have been designed for use inside many areas of the human body. In the cardiovascular system, a common diagnostic ultrasound method is intraluminal ultrasound imaging, with intra-cardiac echocardiography (ICE) being a specific example of intraluminal imaging. Typically, a single rotating transducer or an array of transducer elements is used to transmit ultrasound at the tips of the catheter. The same transducers (or separate transducers) are used to receive echoes from the tissue. A signal generated from the echoes is transferred to a console which allows for the processing, storing, display, or manipulation of the ultrasound-related data.

Intraluminal ultrasound catheters are typically used in the large and small blood vessels (arteries or veins) of the body, and can be delivered over a guidewire having a flexible tip. Intraluminal imaging catheters such as ICE catheters are usually used to image heart and surrounding structures, for example, to guide and facilitate medical procedures, such as transseptal lumen punctures, left atrial appendage closures, atrial fibrillation ablation, and valve repairs. In some cases, commercially-available ICE imaging catheters are not designed to be delivered over a guidewire, but instead have distal ends that can be articulated by a steering mechanism located in a handle at the proximal end of the catheter. For example, an intraluminal imaging catheter such as an ICE catheter may be inserted through the femoral or jugular artery when accessing the anatomy, and steered in the heart to acquire images necessary to the safety of the medical procedures.

An ICE catheter typically includes imaging transducers for ultrasound imaging that generates and receives acoustic energy. The imaging core may include a lined array of transducer elements or transducer elements arranged in any suitable configuration. The imaging core may be encased in an imaging assembly located at a furthest distal tip of the catheter. The imaging assembly may be covered with acoustic adhesive materials. An electrical cable may be soldered to the imaging core and extends through the core of the body of the catheter. The electrical cable may carry control core signals and echo signals to facilitate imaging of the heart anatomy. The assembly may provide rotational, 2-way, or 4-way steering mechanisms such that several views of the heart may be imaged, including, for example, anterior, posterior, left, and/or right views of the heart.

Matrix array ultrasound transducers with substantially square (non-elongated; array with two substantially equal axes/dimensions) have been used for 3D imaging and imaging multiple planes. By applying appropriate phases to the transducer signals, the matrix array may be steered and directed to desired planes to image the desired planes or image a 3D volume.

A problem exists when the ultrasound array is rectangular (elongated; array has a long axis/dimension and a short axis/dimension). The imaging aperture is longer in one dimension and shorter in the other. rectangular/elongated nature of the intraluminal imaging aperture causes different imaging resolutions when imaging multiple planes, such as to acquire a first image spanning the longitudinal axis of the rectangular array along the longer dimension and a second image spanning the transverse axis of the rectangular along the shorter dimension such that the second image is perpendicular to the first image. Also, this problem may create 3D images that have different resolution along different dimensions.

For example, there may be one or more particular views of the anatomy that the user would like to see. However, due to the rectangular/elongated nature of the aperture, the particular views that the user would like to see may have a relatively low resolution.

### SUMMARY

The invention is notably defined by the set of claims. To address these problems, the present disclosure discloses systems, apparatuses, and methods for optimizing (e.g., providing higher resolution for) the anatomical view planes that the user is interested in viewing. For example, the present disclosure provides a method in which a 3D image is captured by a rectangular/elongated array of ultrasound imaging elements while the array is in a first pose. The 3D image is then segmented using model-based segmentation to identify 2D view planes that the user may be interested in viewing. The system (e.g., the processor of the system) may then determine whether one or more of the desired 2D view planes (which may be selected by the user) have a high or low image quality (IQ). If one or more desired 2D view planes have a low IQ, the processor may determine a second pose for the rectangular/elongated array in which the desired 2D view planes would be captured at a higher IQ. The processor may then generate a recommendation for the user that includes a suggested movement to move the rectangular/elongated array so that the desired 2D view planes can be captured at a higher IQ. In this way, 2D images at all of the desired 2D view planes can be obtained with a high enough IQ for the user to sufficiently and effectively analyze the 2D images to, for example, diagnose diseases of the heart or monitor treatment of the heart. Aspects of the present disclosure advantageously ensure that the specific views that the user would like to see are imaged with a high enough resolution for the user to perform the necessary analysis.

One or more embodiments of the present disclosure may include an apparatus, including a rectangular ultrasound array having a long axis and a short axis perpendicular to the long axis and a processor configured for communication with the rectangular ultrasound array. The processor may be configured to control the rectangular ultrasound array to obtain three-dimensional (3D) ultrasound data of an anatomy of a patient while the rectangular ultrasound array is in a first pose. The processor may also be configured to determine a location of a two-dimensional (2D) view plane of the anatomy within a 3D model of the anatomy based on at least the 3D ultrasound data. The processor may also be configured to determine, for the 2D view plane, a first scan angle associated with the first pose of the rectangular ultrasound array. The first scan angle may include an orientation of the 2D view plane relative to at least one of the long axis or the short axis. The processor may also be configured to determine, for the first scan angle, a first image quality associated with the orientation of the 2D view plane relative to at least one of the long axis or the short axis. The processor may also be configured to output, to a display in communication with the processor, a screen display comprising at least one of the location of the 2D view plane, the first scan angle, or the first imaging quality.

In some embodiments, the screen display may include a graphical representation of the rectangular ultrasound array in the first pose, a plurality of image quality regions overlaid on the graphical representation, and an indicator identifying the first scan angle relative to a plurality of image quality regions.

In some embodiments, the processor may be configured to determine, for the 2D view plane, a second scan angle comprising a second image quality that is relatively higher than the first image quality. The screen display may include at least one of the second scan angle or the second image quality. In some embodiments, the processor may be configured to generate user guidance to move the rectangular ultrasound array to a second pose. In the second pose, the 2D view plane may include the second scan angle. The screen display may further include the user guidance. In some embodiments, after movement of the rectangular ultrasound array to the second pose, the processor may be further configured to control the rectangular ultrasound array to_obtain further ultrasound data of the anatomy. In some embodiments, the screen display may further include at least one of a text or a visual representation of the user guidance, a graphical representation of the rectangular ultrasound array in the second pose, a plurality of image quality regions overlaid on the graphical representation, and an indicator identifying the second scan angle relative to plurality of image quality regions. In some embodiments, to generate the user guidance, the processor may be configured to perform a subtraction using a value of the first scan angle and a value of the second scan angle.

In some embodiments, before the determination of the location of the 2D view plane, the processor may be configured to receive a user input identifying the 2D view plane. In some embodiments, before the determination of the location of the 2D view plane, the processor may be configured to output a list of a plurality of 2D view planes. The user input may include selection of the 2D view plane from the list.

In some embodiments, the processor may be configured to determine a location for each of a plurality of 2D view planes within the 3D model, where the 2D view plane is one of the plurality of 2D view planes. In some embodiments, the processor is configured to determine a plurality of first scan angles for the plurality of 2D view planes, determine a plurality of first image qualities for the plurality of first scan angles, output, to the display, a list based on at least one of the location for each of the plurality of 2D view planes, the plurality of first scan angles, or the plurality of first image qualities, and receive a user input selecting the 2D view plane from the list.

In some embodiments, to determine the first image quality, the processor may be configured to determine that the first scan angle extends within one of a plurality of image quality regions. The plurality of image quality regions may include a first image quality region having a first scan angle range proximate to the long axis of the rectangular ultrasound array and a second image quality region having a second scan angle range proximate to the short axis of the rectangular ultrasound array. In some embodiments, the plurality of image quality regions may also include a third image quality region comprising a third scan angle range between the first scan angle range and the second scan angle range.

In some embodiments, the processor may be configured to determine an additional location of an additional 2D view plane of the anatomy within the 3D model of the anatomy based on the 3D ultrasound data. The processor may also be configured to determine, for the additional 2D view plane, an additional first scan angle associated with the first pose of the rectangular ultrasound array, where the additional first scan angle may include an orientation of the additional 2D view plane relative to at least one of the long axis or the short axis. The processor may also be configured to determine, for the additional first scan angle, an additional first image quality associated with the orientation of the 2D view plane relative to at least one of the long axis or the short axis. The processor may also be configured to determine, for the 2D view plane and the additional 2D view plane, a second scan angle and an additional second scan angle, where the second scan angle and the additional second scan angle are within either the first image quality region or the third image quality region. The processor may also be configured to generate user guidance to move the rectangular ultrasound array to a second pose in which the 2D view plane comprises the second scan angle and the additional 2D view plane comprises the additional second scan angle, where the screen display further includes the user guidance.

In some embodiments, to determine the first image quality, the processor may be configured to select a value associated with the first scan angle from continuous range of values associated with a scan angle range extending between the long axis and the short axis. In some embodiments, the processor may be further configured to control the rectangular ultrasound array to obtain 2D ultrasound data in an interleaved manner with the 3D ultrasound data and output, to the display, a plurality of 2D ultrasound images based on the 2D ultrasound data. The processor may be configured to perform the determination of the location of the 2D view plane, the determination of the first scan angle, and the determination of the first image quality while display of the plurality of 2D ultrasound images is ongoing. In some embodiments, the apparatus may further include an intracardiac echocardiography (ICE) catheter and the anatomy of the patient may include a heart of the patient.

One or more embodiments of the present disclosure may include a system including an intraluminal imaging device configured to be advanced through a lumen of a patient and having an array of imaging elements and a processor configured for communication with the intraluminal imaging device. The array of imaging elements may have a rectangular/elongated shape with a long axis and a short axis perpendicular to the long axis. The processor may be configured to control the intraluminal imaging device to acquire a 3D image using the array of imaging elements. The processor may also be configured to generate a patient-specific 3D model using the 3D image and a generic 3D model. The processor may also be configured to identify at least one slice of the patient-specific 3D model that corresponds to at least one standard view. The processor may also be configured to determine a subset of the at least one slice, where each slice of the subset may include an angle in a critical angle range. The processor may also be configured to determine a first at least one of a location or an orientation at which a first angle of a first slice of the subset is in in a non-critical range. The processor may also be configured to output a recommendation to a display, where the recommendation comprises the first at least one location or orientation.

In some embodiments, the recommendation may further include a guidance for moving the intraluminal imaging device to the first at least one location or orientation. In some embodiments, the guidance may include at least one of a rotation or a flexion of a distal portion of the intraluminal imaging device, where the distal portion comprises the array of imaging elements. In some embodiments, the processor may be further configured to acquire a first subsequent image at the first at least one location or orientation. In some embodiments, the first subsequent image may be a 3D image or a two-dimensional (2D) image acquired at the first angle.

In some embodiments, the processor may be further configured to receive a user input selecting the at least one standard view. In some embodiments, the processor may be further configured to automatically select the at least one standard view. In some embodiments, the processor may be configured to generate a patient-specific 3D model using model-based segmentation. In some embodiments, the processor is configured to determine a second at least one of a location or an orientation at which a second angle of a second slice of the subset is in in a non-critical range. In some embodiments, the recommendation may further include the second at least one location or orientation. In some embodiments, the recommendation may further include a first guidance for moving the intraluminal imaging device to the first at least one location or orientation and a second guidance for moving the intraluminal imaging device to the second at least one location or orientation. In some embodiments, the processor is further configured to acquire a first subsequent image at the first at least one of location or orientation and a second subsequent image at the second at least one of location or orientation.

One or more embodiments of the present disclosure may include a method. The method may include the steps of: acquiring a 3D image using an intraluminal imaging device, where the intraluminal imaging device may include an array of imaging elements having a rectangular/elongated shape with a long axis and a short axis perpendicular to the long axis; generating a patient-specific 3D model using the 3D image and a generic 3D model; identifying at least one slice of the patient-specific 3D model that corresponds to at least one standard view; determining a subset of the at least one slice, where each slice of the subset may include an angle in a critical angle range; determining a first at least one of a location or an orientation at which a first angle of a first slice of the subset is in in a non-critical range; and outputting a recommendation to a display, where the recommendation may include the first at least one location or orientation.

In some embodiments, the recommendation may further include a guidance for moving the intraluminal imaging device to the first at least one location or orientation. In some embodiments, the method may further include moving the intraluminal imaging device to the first at least one location or orientation. In some embodiments, moving the intraluminal imaging device may include at least one of a rotation or a flexion of a distal portion of the intraluminal imaging device, where the distal portion may include the array of imaging elements. In some embodiments, the method may further include acquiring a first subsequent image at the first at least one location or orientation. In some embodiments, the method may further include, before acquiring the 3D image, selecting the at least one standard view. In some embodiments, selecting the at least one standard view may include automatically selecting the at least one standard view. In some embodiments, selecting the at least one standard view may include receiving a user selection of the at least one standard view.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
Fig. 1 is a schematic diagram of an intraluminal imaging system, according to aspects of the present disclosure.
Fig. 2 is a perspective view of an imaging assembly, according to aspects of the present disclosure.
Fig. 3 is a top view of a tip member according to aspects of the present disclosure.
Fig. 4 is a schematic diagram of an array of imaging elements capturing a 2D view plane, according to aspects of the present disclosure.
Fig. 5 is a schematic diagram of an array of imaging elements capturing a 3D volume, according to aspects of the present disclosure.
Fig. 6 is a schematic diagram of a rectangular array of imaging elements, according to aspects of the present disclosure.
Fig. 7 is a schematic diagram of a high image quality (IQ) and low IQ regions of the rectangular array of imaging elements, according to aspects of the present disclosure.
Fig. 8A is a schematic diagram of a 2D view plane captured by a rectangular array of imaging elements, according to aspects of the present disclosure.
Fig. 8B is a schematic diagram of another 2D view plane captured by a rectangular array of imaging elements, according to aspects of the present disclosure.
Fig. 9 is a flow chart of a method of capturing 2D images at desired anatomical views with a higher IQ, according to aspects of the present disclosure.
Fig. 10 is an exemplary screen display, according to aspects of the present disclosure.
Fig. 11 is a flow chart illustrating a process of model-based segmentation, according to aspects of the present disclosure.
Figs. 12A-12B is an exemplary 3D mesh model of the heart, according to aspects of the present disclosure.
Figs. 13A-13B is an exemplary adapted 3D mesh model of the heart generated from the 3D mesh model shown in Figs. 12A-12B and 3D imaging data, according to aspects of the present disclosure.
Fig. 14 is an exemplary updated screen display, according to aspects of the present disclosure.
Fig. 15 is an exemplary further updated screen display, according to aspects of the present disclosure.
Fig. 16 is an illustration of a cross-section of the human heart, according to aspects of the present disclosure.
Fig. 17 is a schematic diagram of a tricuspid valve, according to aspects of the present disclosure.
Fig. 18 is an exemplary 3D ultrasound image of a tricuspid valve, according to aspects of the present disclosure.
Fig. 19A is a schematic diagram of a geometrical 3D mesh model of a tricuspid valve, according to aspects of the present disclosure.
Fig. 19B is a schematic diagram of a 3D image of a tricuspid valve, according to aspects of the present disclosure.
Fig. 19C is a schematic diagram of an adapted 3D mesh model of a tricuspid valve generated from the geometrical 3D mesh model in Fig. 19A and the 3D image in Fig. 19B, according to aspects of the present disclosure.
Fig. 20A is a schematic diagram of the geometrical 3D mesh model shown in Fig. 19A with key or relevant view planes of the tricuspid valve, according to aspects of the present disclosure.
Fig. 20B is a schematic diagram of the adapted 3D mesh model shown in Fig. 19C with key view planes of the tricuspid valve, according to aspects of the present disclosure.
Fig. 21A is a schematic diagram of an adapted 3D mesh model of a heart, according to aspects of the present disclosure.
Fig. 21B is a schematic diagram of an adapted 3D mesh model of the tricuspid valve shown in Figs. 19C and 20B, according to aspects of the present disclosure.
Fig. 22 is a flow chart of a method in which the desired 2D view planes are selected after the 3D imaging data has been acquired and model-based segmentation has been performed, according to aspects of the present disclosure.
Fig. 23 is an exemplary screen display, according to aspects of the present disclosure.
Fig. 24 is a schematic diagram illustrating high-IQ regions, low-IQ regions, and medium-IQ regions of a rectangular array of imaging elements, according to aspects of the present disclosure.
Fig. 25 is an exemplary further updated screen display, according to aspects of the present disclosure.
Fig. 26 is a schematic diagram illustrating exemplary continuous quality scores for the rectangular array of imaging elements, according to aspects of the present disclosure.
Fig. 27 is a flow chart illustrating a method for obtaining a 2D image of the selected 2D view planes at the second pose, according to aspects of the present disclosure.
Fig. 28 is a flow chart illustrating a method in which 2D ultrasound images are output to a display while further analysis of the 3D imaging data is ongoing, according to aspects of the present disclosure.
Fig. 29 is a schematic diagram of a processor circuit, according to aspects of the present disclosure.

### DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. For example, while the ICE system is described in terms of intraluminal imaging, it is understood that it is not intended to be limited to this application. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

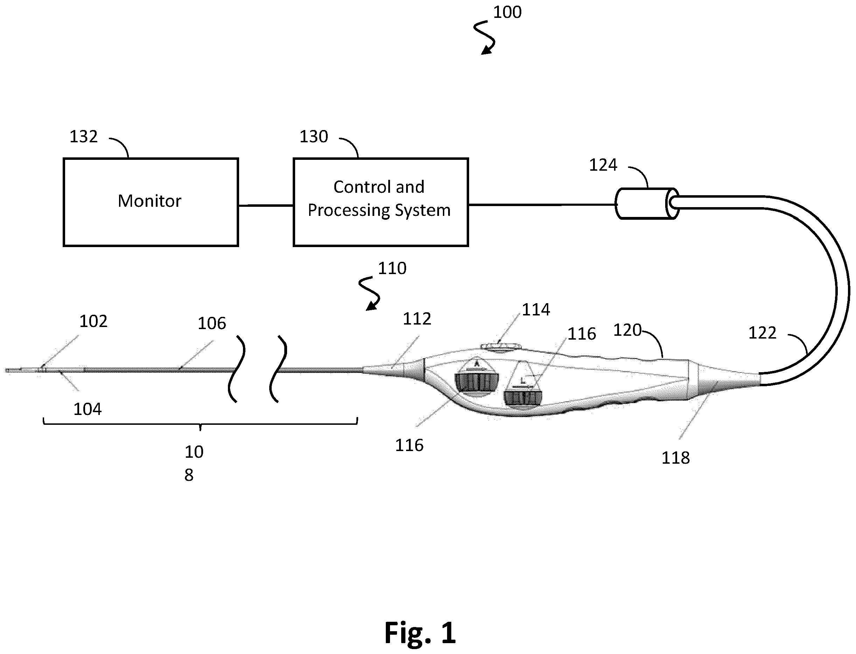

Fig. 1 is a schematic diagram of an intraluminal imaging system 100 according to embodiments of the present disclosure. The system 100 may include an intraluminal imaging device 110, a connector 124, a control and processing system 130, such as a console and/or a computer, and a monitor 132. The intraluminal imaging device 110 includes an imaging assembly 102 at the tip of a flexible elongate member 108, and a handle 120. The flexible elongate member 108 includes a distal portion 104 and a proximal portion 106. The distal end of the distal portion 104 is attached to the imaging assembly 102. The proximal end of the proximal portion 106 is attached to the handle 120 for example, by a resilient strain reliever 112, for manipulation of the intraluminal imaging device 110 and manual control of the intraluminal imaging device 110. The imaging assembly 102 can include an imaging core with ultrasound transducer elements and associated circuitry. The handle 120 can include actuators 116, a clutch 114, and other steering control components for steering the intraluminal imaging device 110, such as deflecting the imaging assembly 102 and the distal portion 104, as described in greater details herein.

The handle 120 is connected to the connector 124 via another strain reliever 118 and a connection cable 122. The connector 124 may be configured in any suitable configurations to interconnect with the control and processing system 130 and the monitor 132 for processing, storing, analyzing, manipulating, and displaying data obtained from signals generated by the imaging core at the imaging assembly 102. The control and processing system 130 can include one or more processors, memory, one or more input devices, such as keyboards and any suitable command control interface device. The control and processing system 130 can be operable to facilitate the features of the intraluminal imaging system 100 described herein. For example, the processor can execute computer readable instructions stored on the non-transitory tangible computer readable medium. The monitor 132 can be any suitable display device, such as liquid-crystal display (LCD) panel or the like.

In operation, a physician or a clinician advances the flexible elongate member 108 into a vessel within a heart anatomy. The physician or clinician can steer the flexible elongate member 108 to a position near the area of interest to be imaged by controlling the actuators 116 and the clutch 114 on the handle 120. For example, one actuator 116 may deflect the imaging assembly 102 and the distal portion 104 in a left-right plane and the other actuator 116 may deflect the imaging assembly 102 and the distal portion 104 in an anterior-posterior plane, as discussed in greater details herein. The clutch 114 provides a locking mechanism to lock the positions of the actuators 116 and in turn the deflection of the flexible elongate member while imaging the area of interest.

The imaging process may include activating the ultrasound transducer elements on the imaging assembly 102 to produce ultrasonic energy. A portion of the ultrasonic energy is reflected by the area of interest and the surrounding anatomy, and the ultrasound echo signals are received by the ultrasound transducer elements. The connector 124 transfers the received echo signals to the control and processing system 130 where the ultrasound image is reconstructed and displayed on the monitor 132. In some embodiments, the processing system 130 can control the activation of the ultrasound transducer elements and the reception of the echo signals. In some embodiments, the control and processing system 130 and the monitor 132 may be part of the same system.

The system 100 may be utilized in a variety of applications such as transseptal lumen punctures, left atrial appendage closures, atrial fibrillation ablation, and valve repairs and can be used to image vessels and structures within a living body. Although the system 100 is described in the context of intraluminal imaging procedures, the system 100 is suitable for use with any catheterization procedure, e.g., ICE. In addition, the imaging assembly 102 may include any suitable physiological sensor or component for diagnostic, treatment, and/or therapy. For example, the imaging assembly can include an imaging component, an ablation component, a cutting component, a morcellation component, a pressure-sensing component, a flow-sensing component, a temperature-sensing component, and/or combinations thereof.

In some embodiment, the intraluminal imaging device 110 includes a flexible elongate member 108 that can be positioned within a vessel. The flexible elongate member 108 has a distal portion 104 and a proximal portion 106. The intraluminal imaging device 110 includes an imaging assembly 102 that is mounted within the distal portion 104 of the flexible elongate member 108.

In some embodiments, the intraluminal imaging system 100 is used for generating two-dimensional (2D) and three-dimensional (3D) images. In some embodiments, the intraluminal imaging system 100 is used for generating x-plane images at two different viewing directions perpendicular to each other. In some embodiments, the x-plane images are at two different viewing directions that are not perpendicular to each other.

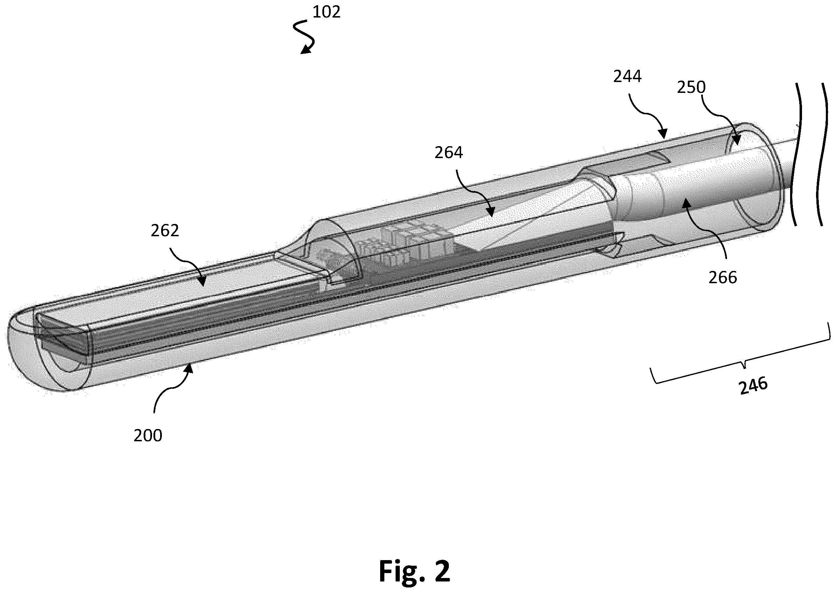

Fig. 2 is a perspective view of the imaging assembly 102 positioned for coupling according to embodiments of the present disclosure. The imaging assembly 102 is illustrated with the imaging core 262 in position within the tip member 200. The imaging core 262 is coupled to the electrical cable 266 via the electrical interconnection 264. The electrical cable 266 extends through the alignment portion 244 and the interface portion 246 of the inner cavity 250. The electrical cable 266 can further extend through the flexible elongate member 108 as shown in Fig. 1.

The configuration and structure of the tip member 200 described above provide several benefits such as safe and easy delivery for catheterization, improved tensile strength for steering or navigation, consistent or automatic alignment, and improved image quality. For example, the outer geometry of the tip member 200 is configured to provide smooth surfaces and smooth edges with small radii. The smooth edges reduce friction when the tip member 200 traverses a vessel during insertion. The smooth surfaces prevent tears and/or damages to tissue structures during the insertion. In addition, the smooth edges and smooth surfaces can facilitate crossing of a septum or other anatomical feature during a catheterization procedure. The material type and the wall thickness of the tip member 200 are selected to minimize acoustic distortion, attenuation, and/or reflection. The internal geometry of the tip member 200 is configured to facilitate alignment during manufacturing. The tip member 200 can also include other features, for example, a guidewire lumen, holes, or other geometry to accommodate additional devices or features such as pressure sensors, drug delivery mechanisms, and/or any suitable interventional features.

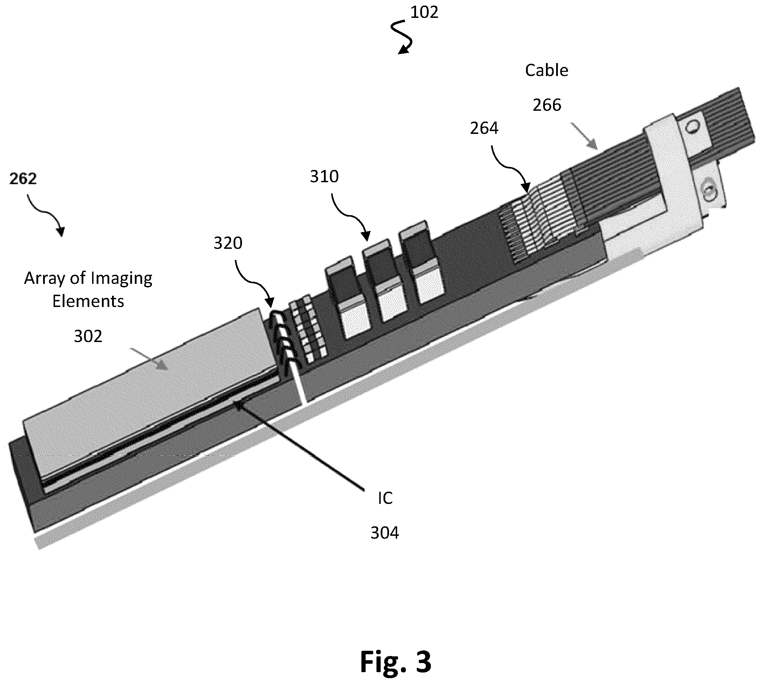

Fig. 3 is a top view of an imaging assembly 102 according to embodiments of the present disclosure. The imaging assembly 102 is illustrated with the imaging core 262 having an array of imaging elements 302 and micro-beamformer IC 304 coupled to the array of imaging elements 302. The imaging assembly 102 also shows the electrical cable 266 coupled to the electrical interconnection 264. In some embodiments, the electrical cable 266 is coupled through an interposer 310 to the micro-beamformer IC 304. In some embodiments the interposer 310 is connected to the micro-beamformer IC 304 through wire bonding 320. In some embodiments, the imaging assembly 102 is configured such that the electrical cable 266 is directly coupled to the micro-beamformer IC 304.

In some embodiments, imaging assembly 102 includes an array of imaging elements 302 in the form of an array of more than 800 imaging elements. The array can be a two-dimensional (2D) array. In some aspects, the array can_be a planar array (e.g., not a curved array). In other aspects, the array can be a curved array. In some embodiments, the array imaging elements 302 is square-shaped or relatively more square-shaped in that it has equal or relatively more equal number of rows of imaging elements and columns of imaging elements (e.g., equal or relatively more equal number of imaging elements in two perpendicular axes such that the two axes are equal or relatively more equal). Relatively more square shaped and relatively more equal can refer to the fact that even if not exactly square or exactly equal, the axes are similar enough so that image quality along both directions is comparable. In some aspects, a square-shaped array can be described as a symmetric/symmetrical aperture. In other aspects, the imaging elements 302 may be arranged in a 2D array having a length greater than width such that more imaging elements 302 extend along the length of the array than across the width (e.g., different number of rows of imaging elements and columns of imaging elements, such that the array has a long axis and a short axis that are not equal). For example, the 2D array may be rectangularly shaped or have an elongated shape. In some aspects, a rectangular/elongated array can be described as having an asymmetric/asymmetrical aperture.

In some embodiments, the array of imaging elements 302 is an array of ultrasound imaging transducers that are directly flip-chip mounted to the micro-beamformer IC 304. The transmitters and receivers of the ultrasound imaging transducers are on the micro-beamformer IC 304 and are directly attached to the transducers. In some embodiments, mass termination of the acoustic elements is done at the micro-beamformer IC 304.

In some embodiments, the micro-beamforming IC 304 lies directly underneath the array of acoustic elements 302 and is electrically connected to them. The array acoustic elements 302 may be piezoelectric or micromachined ultrasonic transducer (MUT) elements. Piezoelectric elements typically would be attached to the IC by flip-chip mounting an assembly of acoustic layers and sawing into individual elements. MUT elements may be flip-chip mounted as a unit or grown directly on top of the micro-beamforming IC 304. In some embodiments, the cable bundle may be terminated directly to the micro-beamforming IC 304, or may be terminated to an interposer 310 of suitable material such as a rigid or flexible printed circuit assembly. The interposer 310 may then be connected to the micro-beamforming IC 304 via any suitable means such as wire bonding 320.

In some embodiments, the micro-beamformer IC 304 can control the array of imaging elements 302 and can perform beam forming for the array imaging elements 302. In some embodiments, the electrical cable 266 further includes one or more power lines for feeding power to the micro-beamformer IC 304, one or more control lines for communicating control signals to the micro-beamformer IC 304, and one or more signal lines for transferring imaging signals.

In some embodiments, the delay elements in use consist of a number of repeated elements, and the number of these elements determines the maximum available delay. Since the acoustic array may be flip-chip mounted to the micro-beamformer IC 304, all of the processing, including the delay, for any given element can reside in the area occupied by that one element.

In some embodiments, a plurality of imaging signals, received by the array of imaging elements, are beam formed. The plurality of imaging signals are associated with a plurality of planes between the first plane and the second plane. A 3D volume image is generated from the plurality of imaging signals such that the 3D image corresponds to a volume image between the first plane and the second plane. The array of imaging elements 302 may be controlled to obtain a 2D image or a 3D image.

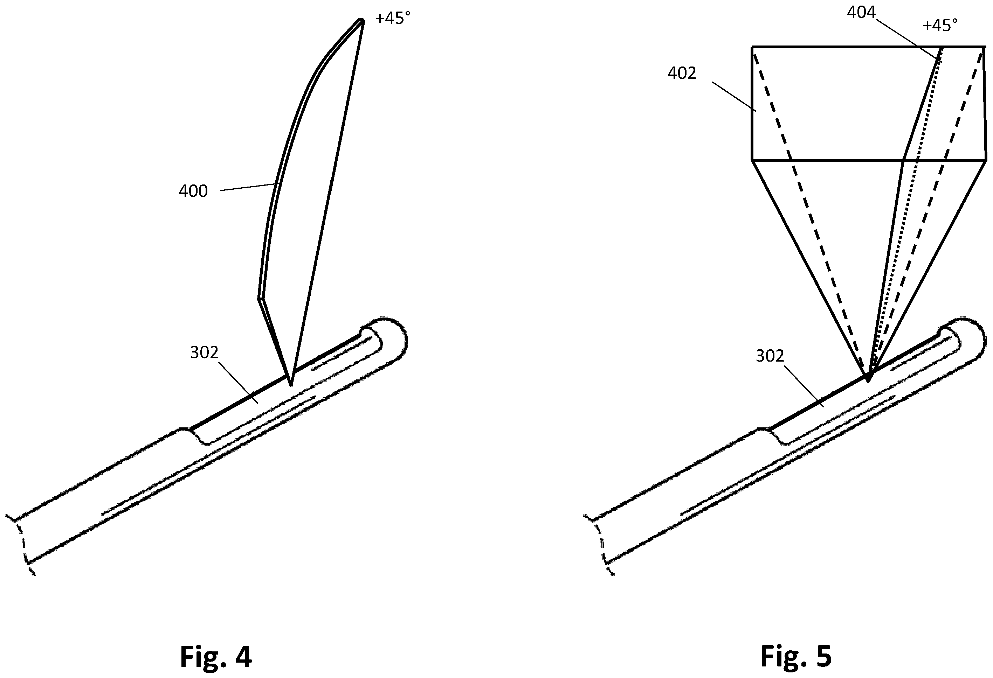

Fig. 4 is a schematic diagram illustrating a 2D imaging view plane 400 captured by the array of imaging elements 302, according to aspects of the present disclosure. The rectangular array of imaging elements 302 can be controlled to electronically steer ultrasound energy to capture 2D ultrasound imaging data along any suitable 2D view plane 400. This 2D ultrasound imaging data may form a 2D ultrasound image. In Fig. 4, the 2D view plane 400 angled at 45 degrees from the axis that extends across the long dimension of the array 302 (i.e. the long axis) and 45 degrees from the axis that extends across the short dimension of the array 302 (i.e. the short axis). However, the array 302 can be electronically steered to acquire imaging data along a 2D view plane at any suitable angle relative to the long and short axes.

Fig. 5 is a schematic diagram illustrating a 3D volume 402 captured by the array of imaging elements 302, according to aspects of the present disclosure. The rectangular array of imaging elements 302 can be controlled to electronically steer ultrasound energy to capture 3D ultrasound imaging data within a 3D volume 402. This 3D ultrasound imaging data may form a 3D ultrasound image. The array 302 can be electronically steered to acquire imaging data within any suitable 3D volume 402.

The diagrams in Figs. 4-5 show a catheter tip consistent with the tip 200 in Fig. 2 and also show an array of imaging elements, which are arranged in a two-dimensional rectangular array. However, any elongated aperture of an imaging array for intraluminal imaging may be used. Examples of other elongated shapes for ultrasound transducer arrays are shown in, e.g., U.S. Patent No. 11,957,513, titled "Non-Rectangular Transducer Arrays And Associated Devices, Systems, And Methods", which is incorporated by reference herein in its entirety.

One or more 2D view planes or slices within the 3D volume may be obtained so that a 2D image can be obtained from the 3D image. For example, as shown in Fig. 5, a 2D view plane 404 angled at 45 degrees from the long axis and 45 degrees from the short axis may be obtained from the 3D volume 402. However, a 2D view plane at any suitable angle relative to the long and short axes may be obtained from the 3D volume. In some embodiments, multiplanar reconstruction (MPR) may be used to obtain one or more 2D imaging planes from the 3D volume.

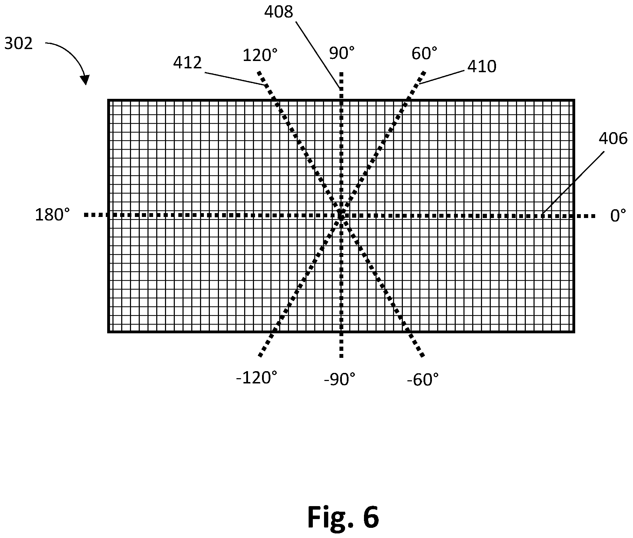

Fig. 6 is a schematic diagram illustrating a rectangular array of imaging elements 302, according to aspects of the present disclosure. The rectangular/elongated array can be part of imaging device, such as an intraluminal catheter (e.g., an ICE catheter, a transesophageal echocardiography or TEE probe, or other intra-body imaging device) or external imaging probe (e.g., a transesophageal echocardiography or TTE probe). The long axis 406 extends along the long dimension (i.e. length) of the array 302 and the short axis 408 extends along the short dimension (i.e. width) of the array 302. The array is not square-shaped because the long and short axes are different from one another. The long axis is physically larger than the short axis and vice versa. The long axis has relatively greater number/quantity of ultrasound transducer elements. The short axis has a relatively fewer number/quantity of ultrasound transducer elements. The long axis 406 and short axis 408 may form a coordinate system within which 2D view planes or 3D volumes can be obtained. For example, as in the illustrated embodiment, the long axis 406 may form a horizontal axis where 0 degrees is marked from the right of the axis and 180 degrees is marked from the left of the axis. The short axis 408, which is perpendicular to the long axis 406, forms the vertical axis where +90 degrees is marked from the top of the axis and -90 degrees is marked from the bottom of the axis. Accordingly, any 2D view plane captured using the array 302 may be described according to this coordinate system. For example, a 2D view plane 410 at 60 or -120 degrees may be obtained. In another example, a 2D view plane 412 at 120 or -60 degrees may be obtained. 2D view planes may also be obtained along the long axis 406 or the short axis 408.

When MPR is used to acquire planes that do not intersect with the array, the dominant direction along the array can be calculated. Because 2D planes including orientation are defined by two axes in space, the axis of the 2D plane that has a smaller component along the normal direction of the matrix (i.e. the larger component parallel to the plane of the matrix) may be determined. Then, this axis may be projected onto the plane of the matrix to determine the effective scanning direction of the 2D plane.

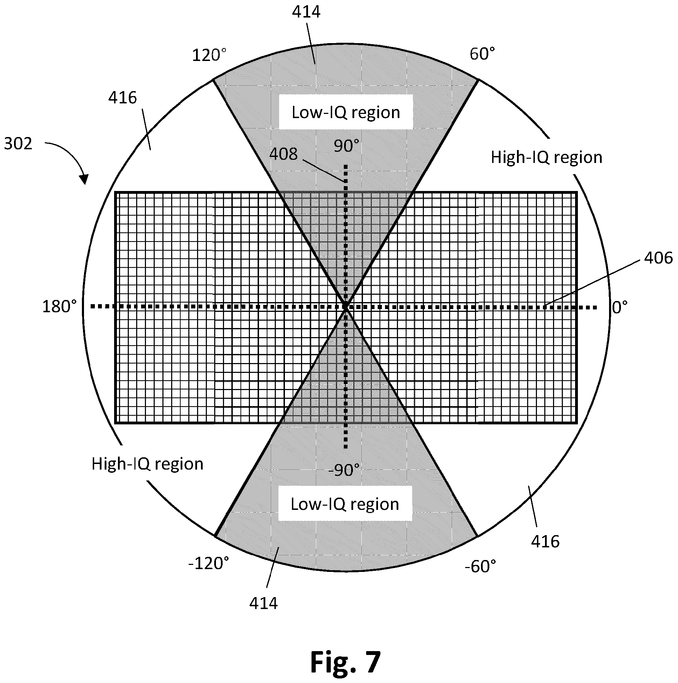

Fig. 7 is a schematic diagram illustrating high image quality (IQ) 416 and low IQ 414 regions of the rectangular array of imaging elements 302, according to aspects of the present disclosure. Because of the rectangular/elongated shape of the array 302, the IQ of the 2D image acquired along a 2D view plane may vary depending on the angle relative to the long 406 and short 408 axes. For example, because the array of imaging elements 302 is shorter along the short axis 408 (e.g., contains relatively smaller quantity of imaging elements) than the long axis 406 (along which the array 302 contains more imaging elements), the IQ of a 2D image acquired along the short axis 408 or proximate thereto may be lower than the IQ of a 2D image acquired along the long axis 406 (e.g., contains relatively greater quantity of imaging elements). Thus, low-IQ regions 414 may be defined about the short axis 408 in which the IQ of images captured along view planes in these regions is low (which may also be referred to as a critical angle range). For example, a low-IQ region 414 may extend from 60 degrees to 120 degrees from the long axis 406 such that the low-IQ region 414 includes the short axis 408 at 90 degrees from the long axis 406. Additionally, another low-IQ region 414 may extend from -120 degrees to -60 degrees from the long axis 406 such that the low-IQ region 414 includes the short axis 408 at -90 degrees from the long axis 406. In some embodiments, high-IQ regions may be defined about the long axis 406 in which the IQ of images captured along view planes in these regions is high (which may also be referred to as a non-critical angle range). For example, a high-IQ region 416 may extend from -60 degrees to 60 degrees from the long axis 406 such that the high-IQ region 416 includes the long axis 406 at 0 degrees. Additionally, another high-IQ region 416 may extend from 120 degrees to -120 degrees from the long axis 406 such that the high-IQ region 416 includes the long axis 408 at 180 degrees.

These low-IQ 414 and high-IQ 416 regions are exemplary. Any suitable low-IQ or high-IQ regions may be used. In some embodiments, the low-IQ and high-IQ regions may be defined based on the particular structure of the array 302. In some embodiments, the low-IQ and high-IQ regions may be defined based on user's tolerance for the resulting IQ. For example, if the user has a low tolerance for low-IQ images, the low-IQ region may be smaller. If the user has a high tolerance for low-IQ images, the low-IQ region may be larger. In some embodiments, the low-IQ and high-IQ regions may be defined based on the system processing or display capabilities.

The IQ of an image may be defined in any suitable way. In some embodiments, the IQ of an image may be based on the image resolution. Thus, images with lower resolution would be defined as low-IQ images and images with a higher resolution would be defined as a high-IQ image. Thus, because fewer imaging elements are disposed along the short axis or proximate thereto than along the long axis or proximate thereto, the resolution of the images collected along view planes proximate to the short axis may be lower compared to the resolution of images collected along view planes proximate to the long axis. In some embodiments, the IQ of an image may be based on the image contrast. Thus, images with lower contrast would be defined as low-IQ images and images with a higher contrast would be defined as a high-IQ image. Thus, because fewer imaging elements are disposed along the short axis or proximate thereto than along the long axis or proximate thereto, the contrast of the images collected along view planes proximate to the short axis may be lower compared to the contrast of images collected along view planes proximate to the long axis.

Aspects related to imaging with a rectangular/elongated array are described, for example, in U.S. Patent No. 12,029,610, titled "X-Plane And 3D Imaging For Asymmetric Apertures" and U.S. Publication No. 2021/0321986, titled "Imaging Plane Control And Display For Intraluminal Ultrasound, And Associated Devices, Systems, And Methods", which are incorporated by reference herein in their entireties.

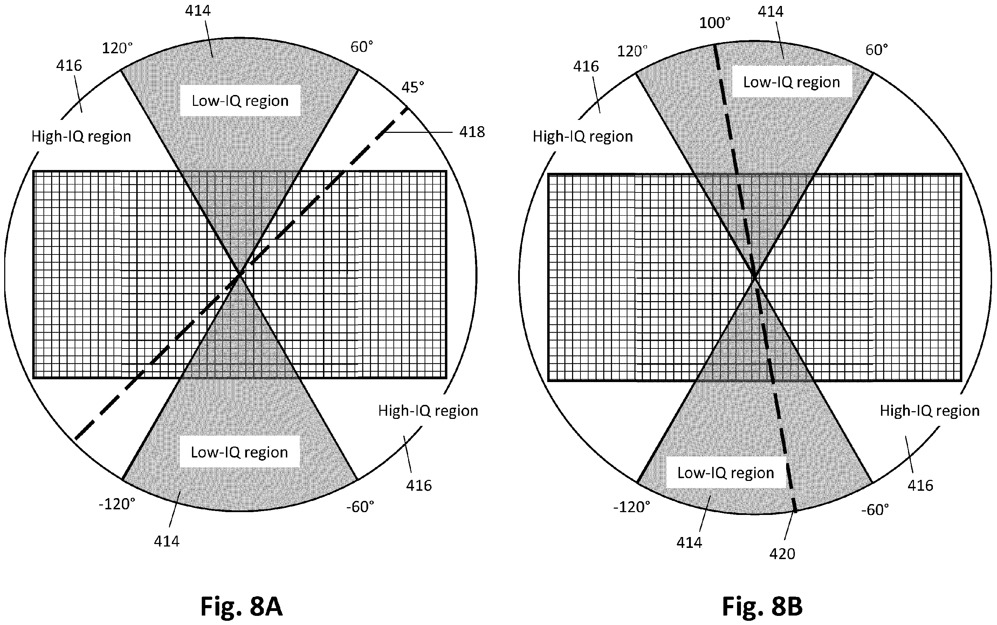

Fig. 8A is a schematic diagram illustrating a 2D view plane 418 captured by a rectangular array of imaging elements 302, according to aspects of the present disclosure. In the illustrated embodiment, the 2D view plane 418 is angled at 45 degrees, which is in the exemplary high-IQ region that ranges from -60 degrees to 60 degrees. Thus, a 2D image acquired along this 45-degree 2D view plane 418 would have a high-IQ (e.g. a high resolution). Note, the 2D view plane 418 may also be defined as being angled at -135 degrees.

Fig. 8B is a schematic diagram illustrating a 2D view plane 420 captured by a rectangular array of imaging elements 302, according to aspects of the present disclosure. In the illustrated embodiment, the 2D view plane 420 is angled at 100 degrees, which is in the exemplary low-IQ region that ranges from 60 degrees to 120 degrees. Thus, a 2D image acquired along this 100-degree 2D view plane 420 would have a low-IQ (e.g. a low resolution). Note, the 2D view plane 420 may also be defined as being angled at -80 degrees.

When capturing a 3D volume of 3D imaging data, the 2D view planes (or slices) of the 3D volume are disposed in a low-IQ region or a high-IQ region depending on their angular position within the defined coordinate system. In some embodiments, a user may not need to view every 2D view plane of the anatomy imaged in the 3D volume. Instead, the user may only be interested in one or more particular views of the anatomy. Thus, a 3D model of the anatomy may be generated based on the 3D imaging data captured by the rectangular array of imaging elements. Then, 2D view planes associated with the particular view of the anatomy may be determined. However, some of the desired 2D view planes may have an angular position that is within a low-IQ region, resulting in a 2D image having a low IQ (e.g. low resolution). For this reason, it may be difficult for the user to see and/or analyze the structure captured in the desired anatomical view. Accordingly, a method that advantageously allows these desired anatomical views to be imaged with a higher IQ is provided.

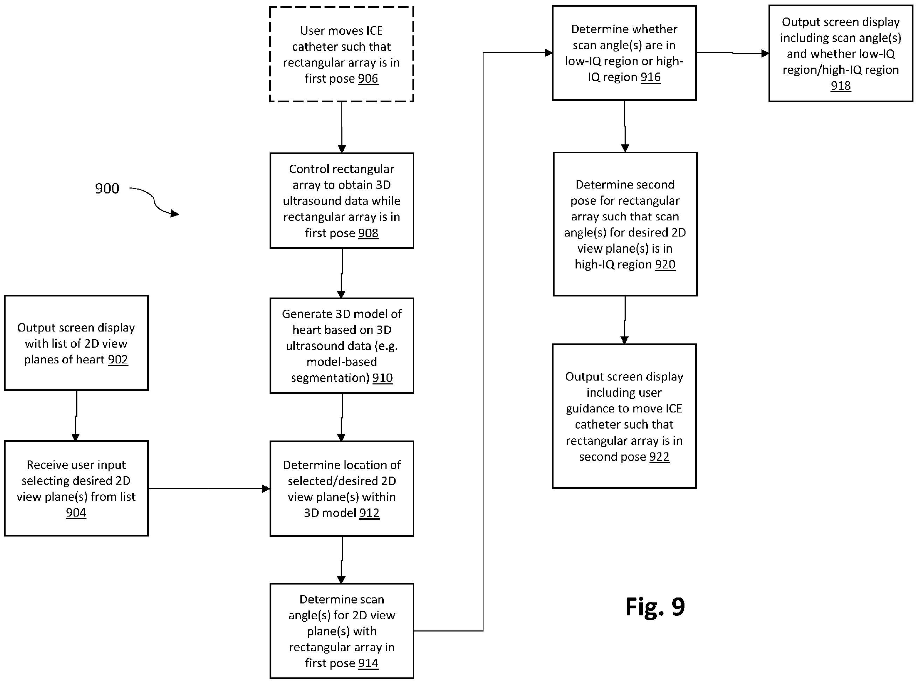

Fig. 9 is a flow chart illustrating a method of capturing 2D images at desired anatomical views with a higher IQ, according to aspects of the present disclosure. In other words, method 900 optimizes the IQ of the 2D images acquired at desired 2D view planes. Although the method 900 is described with reference to an intracardiac echocardiography (ICE) catheter imaging the heart, it should be understood that the method 900 can be applied to any intraluminal imaging device or external imaging device having an asymmetrical aperture. For example, the method 900 may be applied to a transesophageal echocardiogram (TEE) probe or any other endoluminal or endocavity probe. In another example, the method 900 may be applied to a transthoracic echocardiogram (TTE) probe or any other external probe.

Step 902 of method 900 may include outputting a screen display with a list of 2D view planes of the heart. In some embodiments, the screen display may be output to a display coupled to or in communication with the monitor 132 shown in Fig. 1. In some embodiments, the control and processing system 130 shown in Fig. 1 may generate the screen display.

In some embodiments, the 2D view planes listed on the screen display may include standard view planes of the heart. In some embodiments, the screen display may include a list of procedure-related or device-related views. These views may be specific to the procedure or device that is being imaged by the array 302. For example, for a valve repair or replacement procedure, the 2D view planes may be specific to the tricuspid valve or the mitral valve, as described in more detail below. In some embodiments, the 2D view planes may include various angular positions of available 2D view planes.

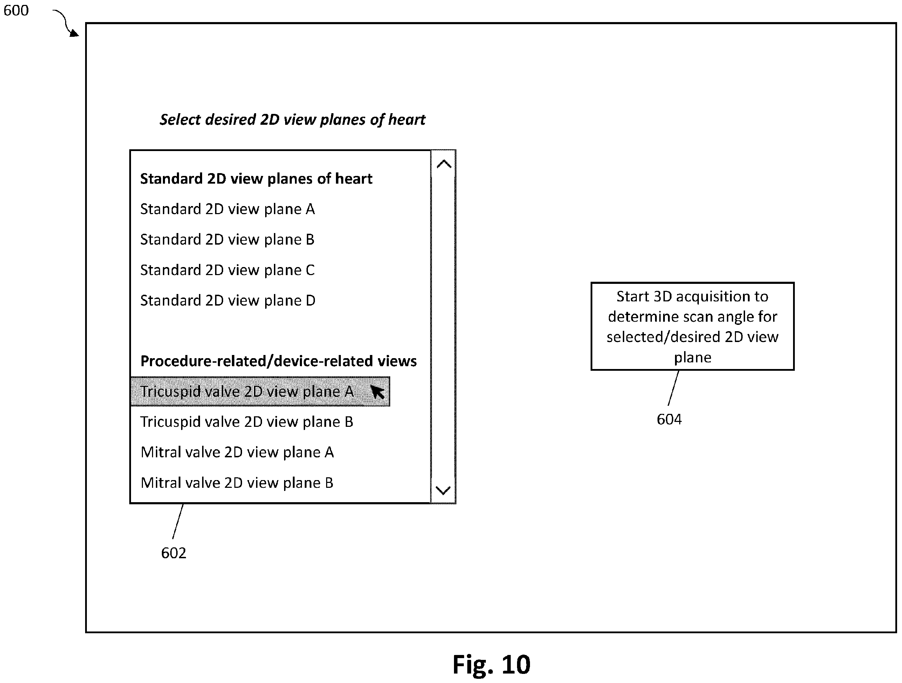

Fig. 10 is an exemplary screen display 600, according to aspects of the present disclosure. In some embodiments, the screen display 600 may include a field 602 including the list of 2D view planes. In the illustrated embodiment, these 2D view planes are grouped by type. In the illustrated embodiment, these types are standard 2D view planes of the heart and procedure-related/device-related views. Example of procedure-related/device-related views include heart valve procedures and/or include heart valve devices, such as for treatment, repair, replacement, etc., of the heart valve. The field 602 can include views related to a commercially available heart valve device, such as a clip (e.g., a medical device that holds two or more valve leaflets together to treat a condition of the heart/heart valve), an artificial valve (e.g., a medical device that replaces a natural heart valve), etc. For example, a manufacturer of the commercially-available heart valve device, a manufacturer of an imaging device (e.g., an ICE catheter, a TEE probe, a TTE probe, and/or other ultrasound probe) used during positioning/deployment of the heart valve device, physicians or professional associations of physicians who perform procedures with the heart valve devices, etc., may recommend that one or more views are useful when positioning/deploying the heart valve device. Such view(s) can be included in the field 602. However, any suitable type of 2D view plane can be listed, including, for example, the angular position of the 2D view planes, the anatomy/anatomies visible in the 2D view plane. Moreover, in some cases, the 2D view planes are listed without dividing them by the type. Each 2D view plane listed is selectable by the user. In some embodiments, the user may select one or more 2D view planes. Once the desired 2D view planes are selected, the user may press or select a button or field 604 that directs the system to start the 3D acquisition of the 3D volume to determine the scan angle for the selected or desired 2D view planes. In some embodiments, the button is not located on the screen display but is instead a physical button.

Returning to Fig. 9, step 904 of the method 900 may include receiving the user input selecting one or more desired 2D view plane from the list. The user may select the desired 2D view planes using any suitable input device. In some embodiments, the input device may be a keyboard, mouse, touchscreen, joystick, button, or any other suitable device. The user may then use the input device to select a button directing the system to start the 3D acquisition of the 3D volume. In some embodiments, the control and processing system 130 shown in Fig. 1 may receive the selected 2D view planes and the direction or instruction to start the 3D acquisition.

Step 906 of the method 900 may include the user moving the ICE catheter such that the rectangular array is in a first pose. This step could be performed during or after steps 902-904. The user may insert the ICE catheter into a vessel of a patient and may be moved through the vasculature such that it is disposed within the heart. For example, the ICE catheter may be moved into the right atrium, right ventricle, left atrium, or left ventricle. In some embodiments, when the system 100 shown in Fig. 1 is used, the flexible elongate member 108 may be inserted into the vasculature of the patient until the imaging assembly 102 having the rectangular array is disposed within the heart. The user may steer the catheter using one or more of the controls within the handle 120, including the actuators 116 and/or the clutch 114.

The pose of the rectangular array may include a position and orientation of the rectangular array. The position may be defined using a 3D coordinate system, such as a coordinate system defined by x-, y-, and z-axes. These axes may be defined based on the device or an external location. The orientation of the device may be defined as the pitch, roll, and yaw of the rectangular array about the three axes (e.g. x-, y-, and z- axes). Thus, the pose of the rectangular array may be defined by six degrees of freedom (including the position and orientation).

In some embodiments, the ICE catheter may be controlled by the control and processing system 130 through the vasculature of the patient until it is in the heart and/or may be controlled by the control and processing system 130 to move the rectangular array to the first pose.

Step 908 of the method 900 may include controlling the rectangular array to obtain 3D ultrasound data while the rectangular array is in the first pose. This step could be performed during or after steps 902-904. However, in a particular embodiment, step 908 is performed after the user selects the desired 2D view planes and directs the system to start the 3D acquisition in step 904.

In some embodiments, the micro-beamformer IC 304 shown in Fig. 1 can command the array imaging elements 302 shown in Fig. 1 and can transmit and receive signals, e.g., ultrasound signals. The micro-beamformer IC 304 can also include a plurality microchannels delay lines. The micro-beamformer IC 304 can supply the required delays for beamforming from one or more of the microchannels delay lines to the array of imaging elements 302. In some embodiments, the beamforming is performed during both transmitting and receiving. In some other examples, the beamforming is performed during the receiving. In some embodiments, the ultrasound signals received by the imaging elements are beamformed by applying the required delays to construct a beam-formed signal associated with the 3D volume.

Accordingly, in some embodiments, the micro-beamformer IC can direct the rectangular array to obtain image data over the 3D volume (i.e. 3D imaging data) in the first pose. In some embodiments, the 3D imaging data is acquired by successively scanning 2D view planes across the 3D volume. In some embodiments, this includes successively activating imaging elements along each 2D view plane. However, the 3D imaging data may be acquired in any suitable way.

Step 910 of the method 900 may include generating a 3D model of the heart based on the 3D ultrasound data.

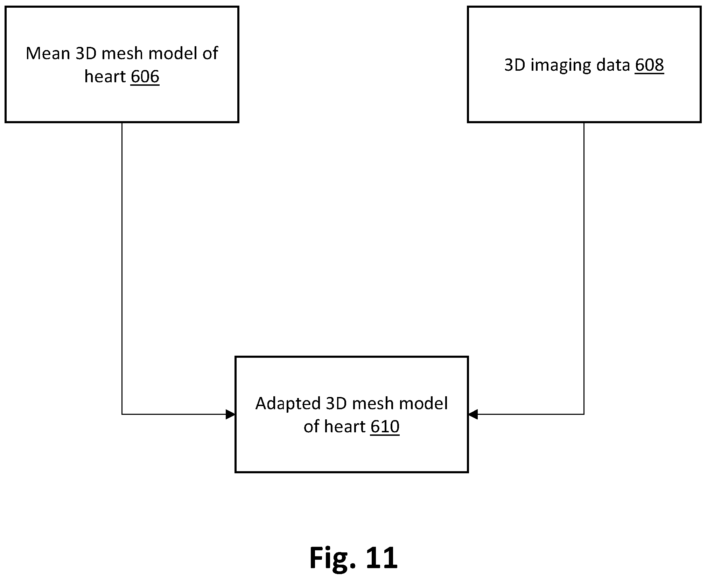

In some embodiments, this may be performed via model-based segmentation. Fig. 11 is a flow chart illustrating (at a high level) a process of performing model-based segmentation, according to aspects of the present disclosure. Model-based segmentation may be done by finding a best match between the 3D imaging data 608 (e.g. 3D ultrasound data) and a geometrical mesh model 606 (i.e. mean 3D mesh model) of the anatomical object of interest (e.g. the heart). Thus, an adapted 3D mesh model 610 of, for example, the heart is generated from the mean 3D mesh model 606 and the 3D imaging data 608. The model-based segmentation may, for example, be conducted in a similar manner as this is described for a model-based segmentation of CT images in Ecabert, O. et al.: "Automatic Model-based Segmentation of the Heart in CT Images", IEEE Transactions on Medical Imaging, Vol. 27(9), p. 1189-1291, 2008, the entirety of which is incorporated herein by reference. Model-based segmentation is also described in, for example, U.S. Patent No. 11,540,718, U.S. Patent No. 11,510,651, U.S. Patent No. 10,424,044, U.S. Patent No. 10,993,700, U.S. Patent No. 11,100,665, U.S. Patent No. 9,956,046, U.S. Patent No. 10,282,846, and U.S. Patent No. 10,402,970, the entireties of which are incorporated herein by reference.

The geometrical mesh model 606 of the anatomical object of interest may comprise respective segments representing respective anatomic features. Accordingly, the control and processing system 130 shown in Fig. 1 may provide an anatomy-related description of the volume data, which identifies respective geometrical locations of respective anatomic features in the volume data.

Such a model-based segmentation usually starts with the identification of the orientation of the anatomical object of interest (e.g. the heart) within the 3D imaging data 608. This may, for example, be done using a 3D implementation of the Generalized Hough Transform. Pose misalignment may be corrected by matching the geometrical model 606 to the image 608 making use of a global similarity transformation. The segmentation comprises an initial model that roughly represents the shape of the anatomical object of interest. Said model may be a multi-compartment mesh model. This initial model will be deformed by a transformation. This transformation is decomposed into two transformations of different kinds: a global transformation that can translate, rotate or rescale the initial shape of the geometrical model, if needed, and a local deformation that will actually deform the geometrical model so that it matches more precisely to the anatomical object of interest. This is usually done by defining the normal vectors of the surface of the geometrical model to match the image gradient; that is to say, the segmentation will look in the received ultrasonic image for bright-to-dark edges (or dark-to-bright), which usually represent the tissue borders in ultrasound images, i.e. the boundaries of the anatomical object of interest.

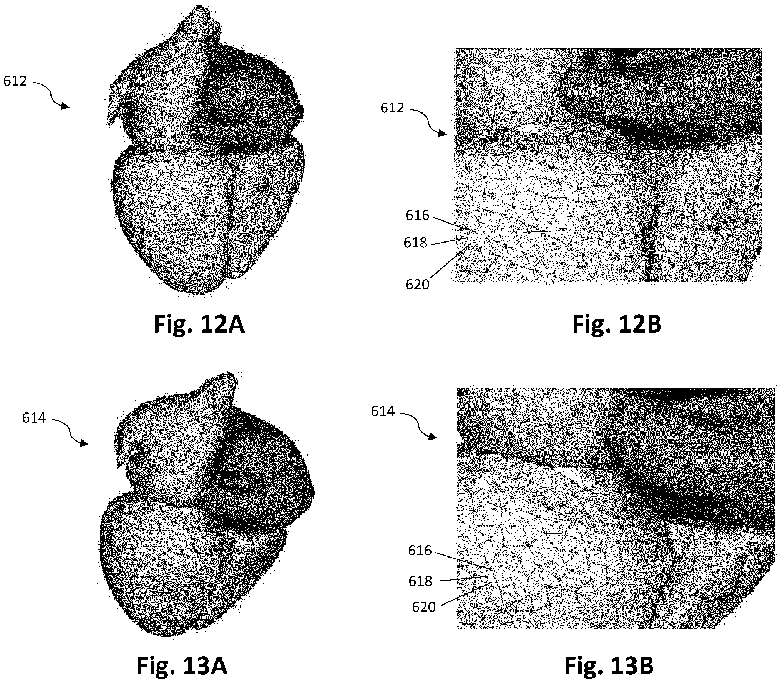

Figs. 12A-12B illustrates an exemplary 3D mesh model of the heart, according to aspects of the present disclosure. Fig. 12A is a perspective view of the whole 3D mesh model 612 of the heart and Fig. 12B is a zoomed-in view of a portion of the 3D mesh model 612 in Fig. 12A. The 3D mesh model includes different sections that correspond to various structures of the heart, including the right atrium, right ventricle, left atrium, and left ventricle. The 3D mesh model also includes a plurality of vertices 616 and edges 618 that extend between neighboring vertices 616. In some embodiments, these vertices 616 and edges 618 form a plurality of surfaces 620. In some embodiments, the surfaces 620 are triangularly shaped.

Figs. 13A-13B illustrates an exemplary adapted 3D mesh model 614 of the heart generated from the 3D mesh model 612 shown in Figs. 12A-12B and 3D imaging data, according to aspects of the present disclosure. Fig. 13A is a perspective view of the whole adapted 3D mesh model 614 of the heart and Fig. 13B is a zoomed-in view of a portion of the adapted 3D mesh model 614 in Fig. 13A. To form the adapted 3D mesh model 614, the vertices 616, edges 618, and surfaces 620 in the generic 3D mesh model 612 shown in Figs. 12A-12B have been transformed to match or fit the 3D imaging data. This transformation may be performed as described above in reference to Fig. 11.

Returning to Fig. 9, step 912 of the method 900 may include determining the location of the selected or desired 2D view planes within the 3D model (e.g. the adapted 3D mesh model generated via model-based segmentation).

In embodiments in which model-based segmentation is used, landmarks may be encoded within the geometrical 3D mesh model. These landmarks may define 2D view planes. A set of three or more landmarks can represent a 2D view plane. These encoded landmarks may be mapped onto the adapted 3D model so as to obtain a set of 2D view planes of the anatomical object of interest generated from the 3D imaging data. In some embodiments, the landmarks encoded in the geometrical 3D mesh model are moved along with the mesh (i.e. vertices, edges, and surfaces) when the geometrical 3D mesh model is adapted to the 3D image. For example, these 2D view planes may be associated with standard 2D view planes or procedure-related/device-related 2D view planes. In some embodiments, the system may determine, for each 2D view plane, anatomical features of the anatomical object of interest that are expected to be contained within the 2D view plane. This may be done using the geometrical model that is encoded with the anatomic features of the anatomical object of interest. It should thus be known which anatomical features should occur in which 2D view planes.

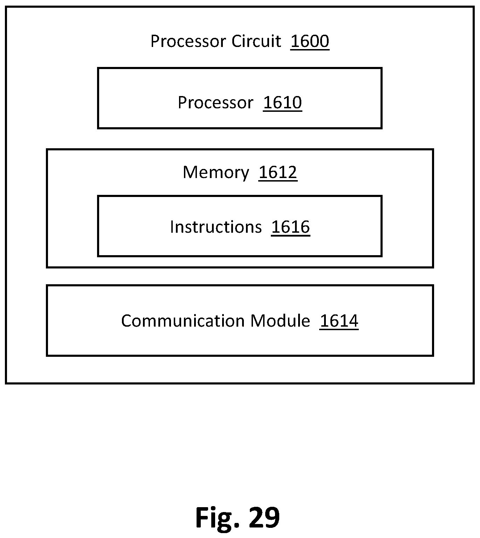

Data representative of the 3D model is stored in memory (e.g., memory 1612 of Fig. 29, e.g., part of processor circuit in system 130 of Fig. 1). In some embodiments, encoding can refer to part of that data (that is representative of 3D model and stored in the memory) is also representative of what is being encoded (e.g., anatomical landmark, location of clip, etc.). In some embodiments, encoding can refer to additional data representative of what is being encoded (e.g., anatomical landmark, location of clip, etc.) that is also stored in the memory. The data representative of what is being encoded can be linked and/or otherwise stored in association with the data representative of the 3D model in the memory.

In some embodiments, the landmarks may be determined using artificial intelligence, such as, for example, neural networks, and, more specifically, convolutional neural networks (CNNs). One or more CNNs may identify one or more landmarks in the 3D ultrasound image or in the adapted 3D model and planes may be generated based on the identified landmarks.

Accordingly, the location of the desired 2D view planes selected by the user within the 3D volume may be determined based on the location of various landmarks or known anatomical structures within the geometric 3D mesh model that is transformed based on the 3D imaging data.

Step 914 of the method 900 may include determining the scan angles (i.e. angular positions) for the selected/desired 2D view planes with the rectangular array in the first pose. In other words, the angular position of the selected 2D view planes in reference to the coordinate system of the rectangular array are determined. For example, the coordinate system of the rectangular array may be that shown in Fig. 6, in which the long axis 406 is defined as 0 degrees from the right and 180 degrees from the left and the short axis 408 is defined as 90 degrees from the top and -90 degrees from the bottom.

Step 916 of the method 900 may include determining whether the scan angles of the selected/desired 2D view planes are in a low-IQ region or a high-IQ region. As described above in reference to Figs. 7-8B, a low-IQ region may be a range of angular positions about the short axis that correspond to scan angles in which the IQ is relatively low (e.g. low resolution) and a high-IQ region may be a range of angular positions about the long axis that correspond to scan angles in which the IQ is relatively high (e.g. high resolution). Thus, to determine whether the selected 2D view planes are in a low-IQ region or a high IQ region, the system (e.g. the control and processing system 130) may compare the angular position of each 2D view plane to the range of angular positions defined as the low-IQ regions and high-IQ regions. For example, the low-IQ regions may be defined as 60 degrees to 120 degrees and -120 degrees to -60 degrees and the high-IQ regions may be defined as -60 degrees to 60 degrees and 120 degrees to -120 degrees (as described above in reference to Figs. 7-8B). Thus, the system may determine which range the angular position of the 2D view plane falls within and thereby determine whether the 2D image associated with the 2D view plane is high-IQ or low-IQ.

In some embodiments, one or more x-planes may be determined for the one or more selected/desired 2D planes. The x-planes may be two 2D planes that are located 90 degrees from each other within the matrix of the rectangular array. In some embodiments, the two 2D planes may be located at another angle from each other such as, for example, 30, 45, 60, or 75 degrees. In some embodiments, the system may automatically define x-planes as being 90 degrees from each other, but the angle may be adjustable by the user or by the system. An example of x-plane imaging is described in U.S. Patent No. 12,029,610, titled "X-Plane And 3D Imaging For Asymmetric Apertures", which is incorporated by reference herein in its entirety.

Step 918 of the method 900 may include outputting a second or updated screen display including, for each selected/desired 2D view plane, the selected/desired 2D view plane, the scan angle, and whether the 2D view plane is in a low-IQ region or a high-IQ region. In some embodiments, the screen display may be output to a display coupled to or in communication with the monitor 132 shown in Fig. 1. In some embodiments, the control and processing system 130 shown in Fig. 1 may generate the screen display. In some embodiments, the updated screen display may be output to the same display as the screen display in step 902.

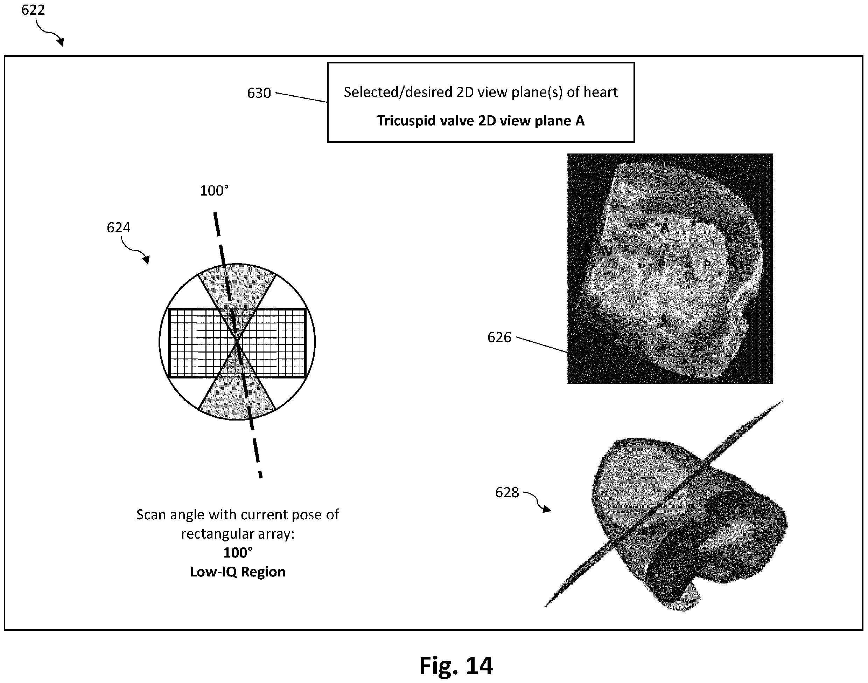

Fig. 14 is an exemplary updated screen display 622, according to aspects of the present disclosure. The updated screen display 622 may include various fields. In some embodiments, the screen display 622 may include a field identifying the selected/desired 2D view plane. In the illustrated embodiment, the 2D view plane selected by the user is the tricuspid valve 2D view plane A. Moreover, in some embodiments, the updated screen display 622 may include a field 624 that shows the scan angle of the selected 2D view plane. In the illustrated embodiment, the system determined that the 2D view plane has a scan angle of 100 degrees, which is within the defined low-IQ region. The field 624 may include the numerical value of the 2D view plane (e.g. 100 degrees) and the IQ region that the 2D view plane is disposed in (e.g. low-IQ region). In some embodiments, field 624 also includes a schematic diagram (e.g. a graphical representation) of the rectangular array and a representation (e.g. dotted line) of the 2D view plane at the determined scan angle relative to the rectangular array. A representation of the low-IQ and high-IQ regions relative to the rectangular array may also be included in field 624. These representations may be overlaid or superimposed over the schematic diagram of the rectangular array so that the user can easily and effectively understand the location of the selected 2D view plane.

In some embodiments, the screen display 622 may also include a field 626 including the 3D image associated with the 3D imaging data at the first pose. In some embodiments, the 3D image is a 3D ultrasound image.

In some embodiments, the screen display 622 may also include a field 628 including the adapted 3D model generated from a generic 3D mesh model and the obtained 3D imaging data in step 910. In other embodiments, field 628 may instead include a 3D image associated with the 3D imaging data obtained in step 908. In some embodiments, a representation of the 2D view plane (e.g. angled 2D plane) relative to the adapted 3D model may be included in field 628. In some embodiments, the representation is overlaid or superimposed over the 3D model so that the user can easily and effectively understand the location of the selected 2D view plane.

When more than one 2D view planes are selected by the user, an additional field (not shown) listing the other selected 2D view planes may also be included in screen display 622. Thus, in some embodiments, only one 2D view plane is shown at a time on the screen display 622. In these embodiments, the user may toggle between each selected 2D view plane to view the information (e.g. scan angle, IQ region) associated therewith. In other embodiments, the information for each selected 2D view plane is included on the same screen display 622.

Returning to Fig. 9, step 920 of the method 900 includes determining a second pose for the rectangular array such that the scan angles for the selected/desired 2D view planes are in the high-IQ regions. In some embodiments, the system may determine a subset of the selected 2D view planes that have a scan angle within a low-IQ region. For this subset of 2D view planes, the 2D images associated therewith have a low IQ (e.g. low resolution) and, thus, the anatomical structures within those images may be difficult for the user to view, see, or analyze. Accordingly, for 2D view planes in the low-IQ regions, the system may determine a second pose at which the 2D view planes are disposed in the high-IQ regions. The second pose may include a second position (e.g. a coordinate in the x-, y-, z-axes) and/or a second orientation (e.g. a pitch, roll, or yaw) of the rectangular array. This second pose would be different from the first pose.

In some embodiments, the system may also determine a guidance for moving the rectangular array from the first pose to the second pose. This guidance may include any suitable movement. For example, the movement may include flexing the distal tip of the ICE catheter including flexing the rectangular array left/right or anterior/posterior, advancing or retracting the ICE catheter that includes the rectangular array into or out of the body, rotating the handle of the ICE catheter towards or away from the user (i.e. clock or counterclock). The present disclosure focuses on movements that physically rotate the rectangular array (e.g. left/right flex); however, it should be known that any suitable movement or combination of movements can be used. The rectangular array can be fixedly coupled to the imaging probe such that movement of the imaging probe causes corresponding movement of the rectangular array. Moreover, in other embodiments, the guidance may include digitally/electronically steering/rotating the plane/volume collected by the rectangular array rather than and/or in combination with manually moving (e.g. rotating) the rectangular array.

In some embodiments, the guidance may include a numerical value representing the extent of the suggested movement. For example, when the suggested movement includes a physical rotation of the rectangular array, the numerical value may be the number of degrees and the direction (e.g. +/-, clockwise/counterclockwise) of the suggested movement. In some embodiments the numerical value may be calculated by subtracting a higher IQ scan angle for a 2D view plane in the second pose from the determined scan angle of the 2D view plane in the first pose.

Step 922 of the method 900 may include outputting a third, further updated screen display including the user guidance to move the ICE catheter such that the rectangular array is in a second pose. In some embodiments, the guidance may include the type of movement and a numerical value indicating the extent and/or direction of the movement.

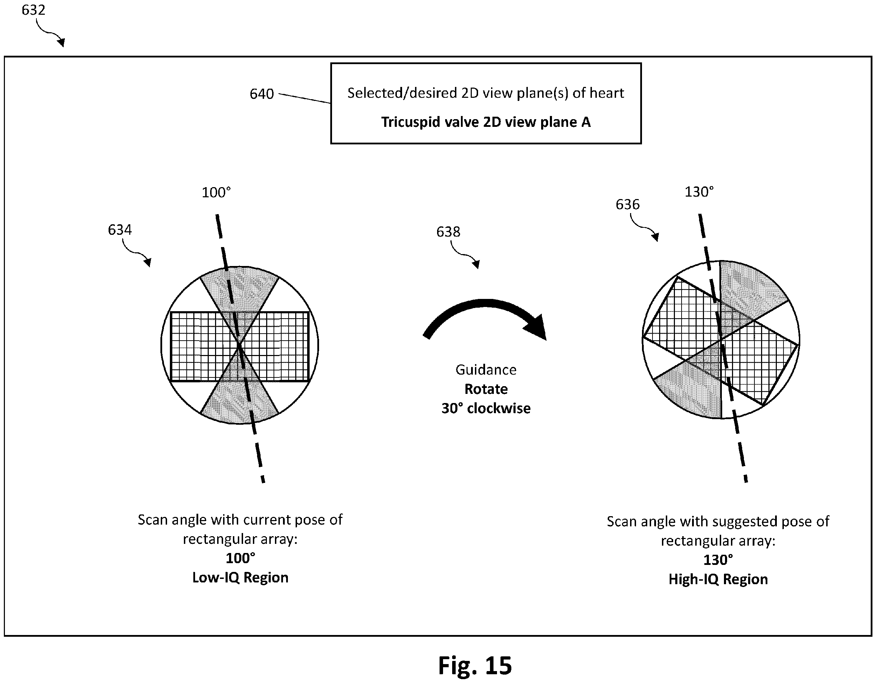

Fig. 15 is an exemplary further updated screen display 632, according to aspects of the present disclosure. The updated screen display 622 may include various fields. In some embodiments, the screen display 622 may include a field identifying the selected/desired 2D view plane. In the illustrated embodiment, the 2D view plane selected by the user is the tricuspid valve 2D view plane A. In some embodiments, this field 640 may be the same as field 630 in Fig. 14.

Moreover, in some embodiments, the further updated screen display 632 may include a field 634 that shows the scan angle of the selected 2D view plane at the first pose (i.e. the first scan angle). In the illustrated embodiment, the first scan angle is 100 degrees, which is within the defined low-IQ region. The field 634 may include the numerical value of the 2D view plane (e.g. 100 degrees) and the IQ region that the 2D view plane is disposed in (e.g. low-IQ region). In some embodiments, field 634 also includes a schematic diagram (e.g. graphical representation) of the rectangular array and a representation (e.g. dotted line) of the 2D view plane at the determined scan angle relative to the rectangular array. A representation of the low-IQ and high-IQ regions relative to the rectangular array may also be included in field 634. These representations may be overlaid or superimposed over the schematic diagram of the rectangular array so that the user can easily and effectively understand the location of the selected 2D view plane. In some embodiments, field 634 is the same as field 624 in Fig. 14.

The further updated screen display 632 may also include a field 638 that includes the user guidance. The user guidance may include a description of the recommended movement (e.g. text describing the movement). For example, in the illustrated embodiment, the recommended movement is to rotate the rectangular array. Moreover, the user guidance may also include a numerical value representing the extent of the recommended movement and/or a direction of the recommended movement. For example, in the illustrated embodiment, the user guidance recommends that the user rotate the rectangular array 30 degrees clockwise. In some embodiments, the numerical value (e.g. 30 degrees) may be calculated by subtracting the higher IQ scan angle of the 2D view plane in the second pose from the determined scan angle of the 2D view plane in the first pose for each 2D view plane. In some embodiments, subtraction may only be performed for one 2D view plane and the second 2D view plane may be determined as being 90 degrees (or any suitable number of degrees) from the first 2D view plane. The user guidance may further include a visual representation (e.g. arrow) associated with the recommended movement.

The further updated screen display 632 may also include a field 636 that shows the scan angle of the selected 2D view plane at the second pose (i.e. the second scan angle). In the illustrated embodiment, the second scan angle is 130 degrees, which is within the defined high-IQ region. The field 636 may include the numerical value of the 2D view plane (e.g. 130 degrees) and the IQ region that the 2D view plane is disposed in (e.g. high-IQ region). In some embodiments, field 636 also includes a schematic diagram (e.g. graphical representation) of the rectangular array and a representation (e.g. dotted line) of the 2D view plane at the determined scan angle relative to the rectangular array. A representation of the low-IQ and high-IQ regions relative to the rectangular array may also be included in field 636. These representations may be overlaid or superimposed over the schematic diagram of the rectangular array so that the user can easily and effectively understand the location of the selected 2D view plane. The schematic diagram of the rectangular array may be rotated relative to that shown in field 634 in accordance with the user guidance.

In some embodiments, steps 920-922 may not be performed if all of the selected/desired 2D view planes are in high-IQ regions. In some embodiments, step 918 may not be performed if one or more of the selected/desired view planes are in low-IQ regions.

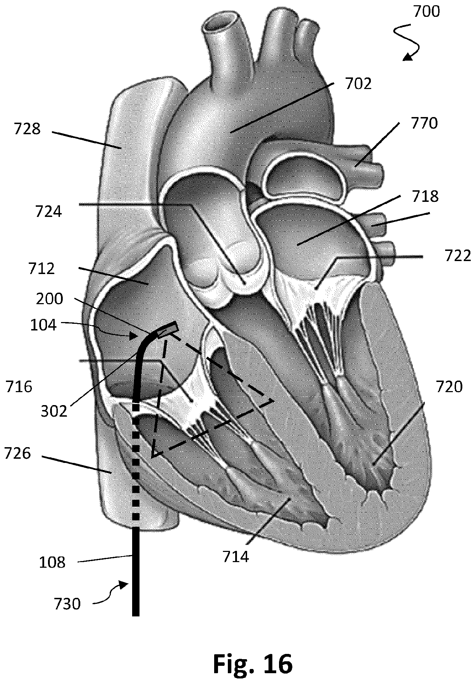

As described above, the method 900 shown in Fig. 9 may use an ICE catheter for imaging the heart or any suitable anatomical structure thereof. In particular, Figs. 16-21B describe an exemplary embodiment in which the user wants to image the tricuspid valve. However, it should be understood that a similar procedure can be used to image any suitable anatomical feature in the heart or other parts of the body.

Fig. 16 is an illustration of a cross-section of the human heart 700, according to aspects of the present disclosure. Visible are a right atrium 712 and a right ventricle 714. In that regard, oxygen-poor blood enters the human heart 700 in the right atrium 712 via the inferior vena cava 726 and the superior vena cava 728 and travels to the right ventricle 714 through the tricuspid valve 716. The oxygen-poor blood leaves the right ventricle 714 and travels to the lungs. Also visible are a left atrium 718 and a left ventricle 720. In that regard, oxygen-rich blood is received from the lungs in the left atrium 718 and travels to the left ventricle 720 through the mitral valve 722. The oxygen-rich blood leaves the left ventricle 720 and goes out to the body through the aorta 702 via an aortic valve 724.

An ICE catheter 730 is disposed within the heart 700. In the illustrated embodiment, the flexible elongate member 108 of the catheter 730 has been snaked through the vasculature such that the tip 200 is disposed within the right atrium 712. Thus, the flexible elongate member 108 may extend through the inferior vena cava 726 into the right atrium 712. The user may move (e.g. advance/retract, flex) the flexible elongate member 108 so that the array of imaging elements 302 is oriented towards the tricuspid valve 716, which may be performed as a part of step 906 in method 900 shown in Fig. 9. For example, the array of imaging elements 302 is an asymmetrical, rectangular array. Accordingly, the rectangular array may be disposed in a first pose from which the tricuspid valve 716 and the surrounding anatomy can be imaged using the rectangular array.

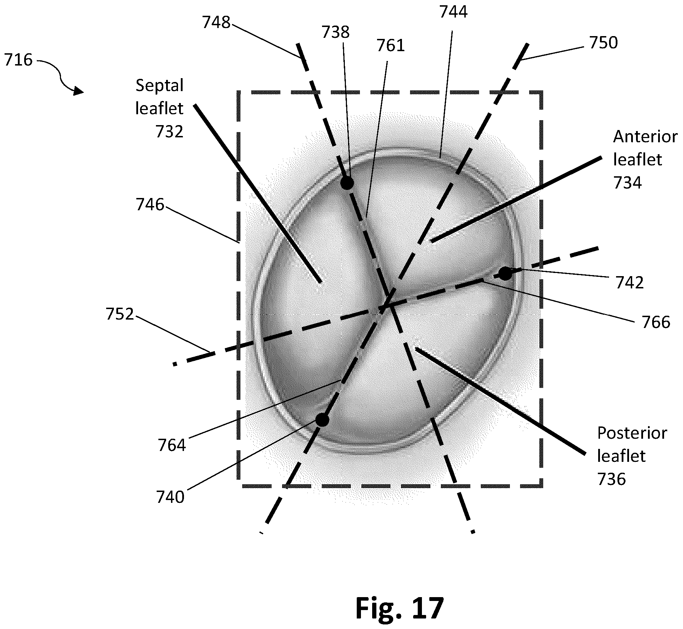

Fig. 17 is a schematic diagram of a tricuspid valve 716, according to aspects of the present disclosure. The tricuspid valve 716 typically includes three leaflets. Standard nomenclature generally refers to these leaflets as the septal leaflet 732, the anterior leaflet 734, and the posterior leaflet 736. These leaflets 732, 734, 736 each open and close to open and close the annulus 744 of the tricuspid valve 716. There are commissures at the points where neighboring leaflets meet: the antero-septal commissure 738 between the anterior leaflet 734 and the septal leaflet 732, the postero-septal commissure 740 between the posterior leaflet 736 and septal leaflet 732, and the antero-posterior commissure 742 between the anterior leaflet 734 and the posterior leaflet 736. Moreover, there are coaptation lines between neighboring leaflets that extend through the commissure therebetween and the opposite leaflet: the antero-septal coaptation line 761 that extends through the antero-septal commissure 738 and the posterior leaflet 736, the postero-septal coaptation line 764 that extends through the postero-septal commissure 740 and the anterior leaflet 734, and the antero-posterior coaptation line 766 that extends through the antero-posterior commissure 742 and the septal leaflet 732.

Thus, for procedures in which imaging of the tricuspid valve 716 is desired (e.g. a tricuspid valve repair or replacement), the user may want to view a 2D image along one or more 2D view planes that show key procedure-specific views of the tricuspid valve 716. For example, these views may include a 2D view plane 746 of the annulus 744. These views may also include a 2D view plane 248 along or parallel to the antero-septal coaptation line 761, which may pass through the antero-septal commissure 738 and the posterior leaflet 736. These views may also include a 2D view plane 750 along or parallel to the postero-septal coaptation line 764, which may pass through the postero-septal commissure 740 and the anterior leaflet 734. These views may also include a 2D view plane 752 along or parallel to the antero-posterior coaptation line 766, which may pass through the antero-posterior commissure 742 and the septal leaflet 732.

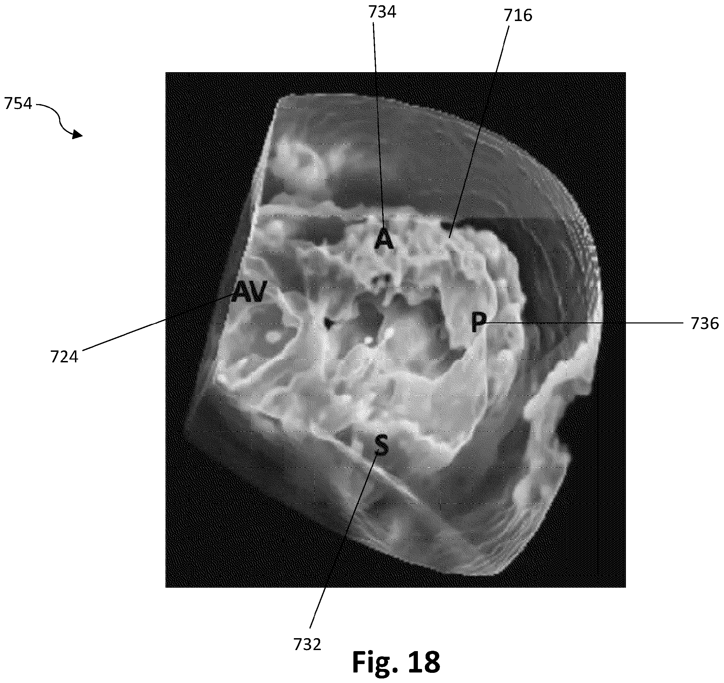

Fig. 18 is an exemplary 3D ultrasound image 754 of a tricuspid valve 716, according to aspects of the present disclosure. In the illustrated embodiment, the septal leaflet 732, anterior leaflet 734, and posterior leaflet 736 can be seen in the 3D ultrasound image 754. Additionally, in the illustrated embodiment, part of the aortic valve 724 can also be seen. In some embodiments, the 3D imaging data may be obtained by the ICE catheter 730 in the first pose shown in Fig. 16, which may be performed as a part of step 908 in method 900 shown in Fig. 9. In some embodiments, the 3D ultrasound image 754 may be acquired during or after a tricuspid valve 716 repair procedure in which a clip is placed within the valve. In some embodiments, the clip is placed between all three leaflets 732, 734, 736 and, in other embodiments, the clip is placed between the septal leaflet 732 and the anterior leaflet 734. Thus, the clip may be captured in the 3D ultrasound image 754 of the tricuspid valve 716.

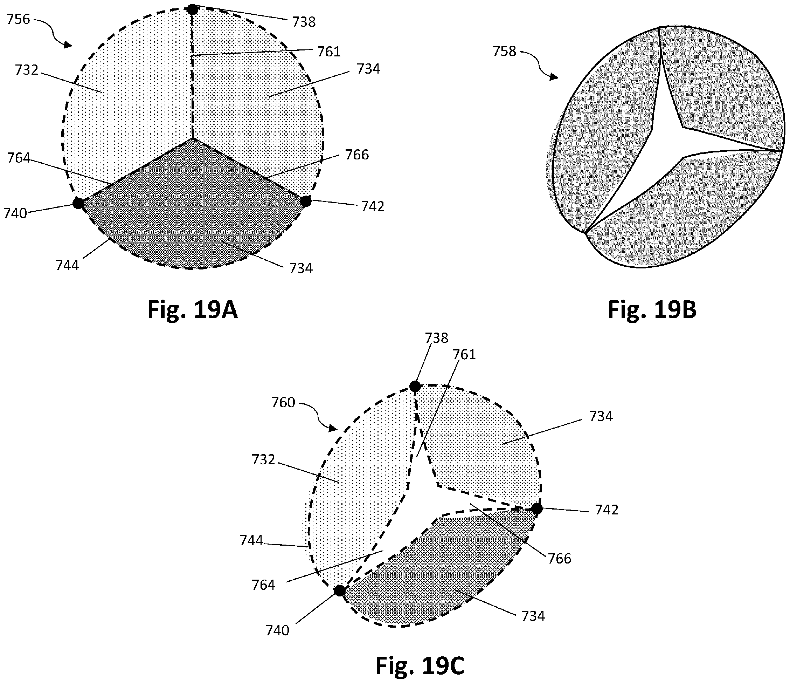

Figs. 19A-19C illustrate applying model-based segmentation to the tricuspid valve 716. Fig. 19A is a schematic diagram of a geometrical 3D mesh model 756 of a tricuspid valve 716. As described above in reference to Figs. 11-13B, the geometrical 3D mesh model may include a plurality of vertices, edges that connect neighboring vertices, and surfaces formed between the edges that outline or define a model of the tricuspid valve 716. In some embodiments, parts of the geometrical 3D mesh model 756 may be associated with different anatomical features of the tricuspid valve. For example, the geometrical 3D mesh model 756 may include parts associated with the septal leaflet 732, the anterior leaflet 734, and the posterior leaflet 736. Moreover, the geometrical 3D mesh model 756 may include parts associated with the antero-septal commissure 738, the postero-septal commissure 740, and the antero-posterior commissure 742. Additionally, the geometrical 3D mesh model 756 may include a part associated with the annulus 744. The geometrical 3D mesh model 756 may also include parts associated with the antero-septal coaptation line 761, the postero-septal coaptation line 764, and the antero-posterior coaptation line 766. In some embodiments, the geometrical 3D mesh model 756 may only include a part for the annulus 744 such that only the annulus 744 of the tricuspid valve 716 is segmented. In these embodiments, other parts of the tricuspid valve 716 (e.g., the septal leaflet 732, the anterior leaflet 734, and the posterior leaflet 736) may be encoded so that the location of these parts can be estimated or determined.

Fig. 19B is a schematic diagram of a 3D image 758 of a tricuspid valve 716. This 3D image 758 may correspond to the 3D imaging data obtained by the ICE catheter 730 in the first pose shown in Fig. 16.

As shown in the illustrated embodiment, the 3D image 758 may not match the geometrical 3D mesh model 756. Thus, the vertices, edges, and surfaces of the geometrical 3D mesh model 756 may be transformed to fit the 3D image 758. This process may generate an adapted 3D mesh model 760. Fig. 19C is a schematic diagram of an adapted 3D mesh model 760 generated from the geometrical 3D mesh model 756 and the 3D image 758. Because the 3D mesh model 756 has been adapted to the 3D image 758 of the tricuspid valve 716, the locations of each of the defined parts of the 3D mesh model 756 have been identified in the 3D image. Thus, model-based segmentation provides information about the different anatomical parts that allows the system to identify the location of parts in the 3D image.

In embodiments in which a clip is included in the 3D image (e.g. during or after a tricuspid valve replacement procedure), the location of the clip may be used when determining the location of the parts of the tricuspid valve 716. For example, if the clip is placed between the septal leaflet 732 and anterior leaflet 734, the system may determine the location of the clip and use this location to determine the location of the septal leaflet 732 and anterior leaflet 734 and/or the location of the desired plane. In some embodiments, the clip is segmented using a geometrical 3D model 756 or another segmentation algorithm (e.g., CNN), but in other embodiments, the location of the clip is encoded.

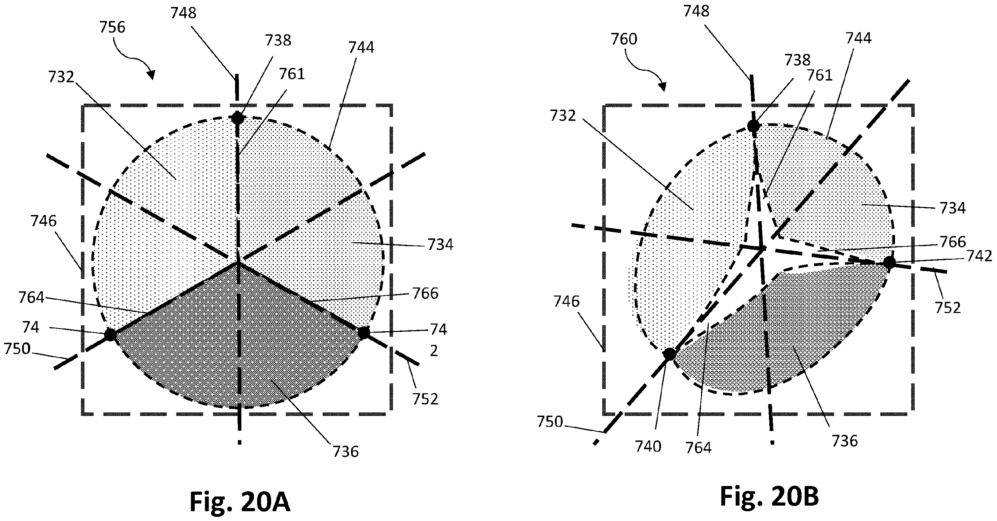

Fig. 20A is a schematic diagram of the geometrical 3D mesh model 756 shown in Fig. 19A with the key or relevant view planes of the tricuspid valve, according to aspects of the present disclosure. In some embodiments, the geometrical 3D mesh model may include or define the key 2D view planes associated with the tricuspid valve. For example, the key views may include a 2D view plane 746 of the annulus 744, a 2D view plane 248 along the antero-septal coaptation line 761 (which may pass through the antero-septal commissure 738 and the posterior leaflet 736), a 2D view plane 750 along the postero-septal coaptation line 764 (which may pass through the postero-septal commissure 740 and the anterior leaflet 734), and a 2D view plane 752 along the antero-posterior coaptation line 766 (which may pass through the antero-posterior commissure 742 and the septal leaflet 732).

Fig. 20B is a schematic diagram of the adapted 3D mesh model 760 shown in Fig. 19C with the key view planes of the tricuspid valve, according to aspects of the present disclosure. In the adapted 3D mesh model 760, the parts of the geometrical 3D mesh model 756 have been adapted to the 3D image and, thus, the key view planes have also been adapted to the 3D image. Accordingly, the location of the key view planes in the adapted 3D mesh model 760 can easily be determined such that the 2D images along the view planes can be generated from the 3D image.

The model-based segmentation described in reference to Figs. 19A-20B may be performed during step 910 of method 900 shown in Fig. 9.

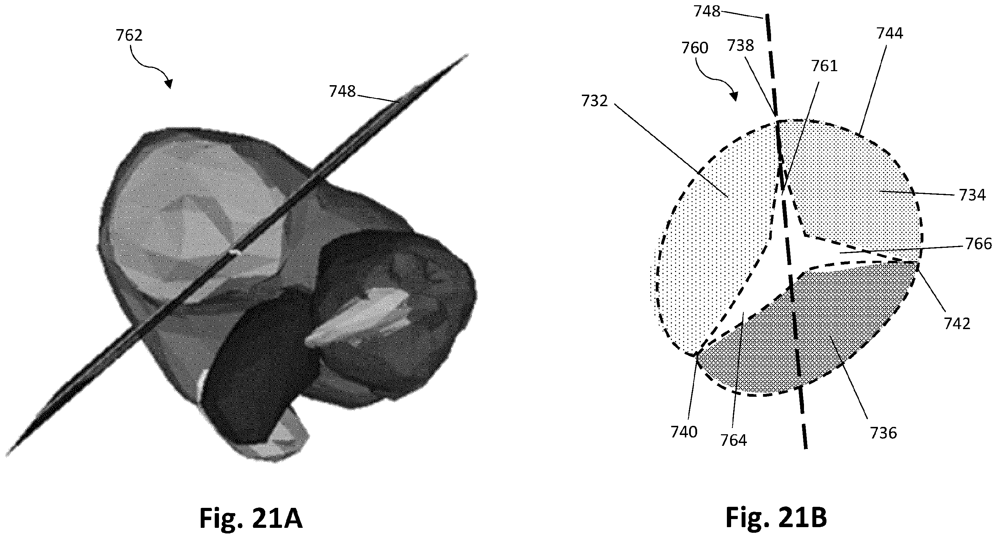

Fig. 21A is a schematic diagram of an adapted 3D mesh model 762 of a heart, according to aspects of the present disclosure. The adapted 3D mesh model 762 of the heart shown in Fig. 21A may include the adapted 3D mesh model 760 of the tricuspid valve shown in Fig. 19B. Thus, the model-based segmentation of the 3D image 758 of the tricuspid valve described in reference to Figs. 19A-20B may be performed during model-based segmentation of the 3D image of the heart. Thus, the key view planes of the tricuspid valve are defined in the adapted 3D mesh model 762 of the heart. Accordingly, any of these key view planes may be selected from within the adapted 3D mesh model 762 of the heart. For example, the 2D view plane 748 along the antero-septal coaptation line 761 may be determined. The illustrated embodiment of the adapted 3D mesh model 762 of the heart illustrates the 2D view plane 748 along the antero-septal coaptation line 761.

Fig. 21B is a schematic diagram of an adapted 3D mesh model 760 of the tricuspid valve shown in Figs. 19C and 20B, according to aspects of the present disclosure. In the illustrated embodiment, the 2D view plane 750 along the antero-septal coaptation line 761 is determined. Thus, Fig. 21B illustrates the same 2D view plane 750 shown in the overall heart adapted 3D mesh model 762 but specifically in the tricuspid valve adapted 3D mesh model 760. As explained above, in some embodiments in which model-based segmentation is performed on a 3D image acquired during a tricuspid valve repair, a clip may be placed between the septal leaflet 732 and the anterior leaflet 734 and the 3D image may capture this clip. Thus, in some embodiments, the user may want to see the 2D plane 748 along the antero-septal coaptation line 761 and the x-plane that is perpendicular to this 2D view plane 248 to view placement of the clip. In some embodiments, the clip may be properly placed when it is perpendicular to the coaptation line 761. For this reason, viewing the x-planes parallel and perpendicular to the antero-septal coaptation line 761 may provide the most useful views for the user to determine proper placement of the clip.

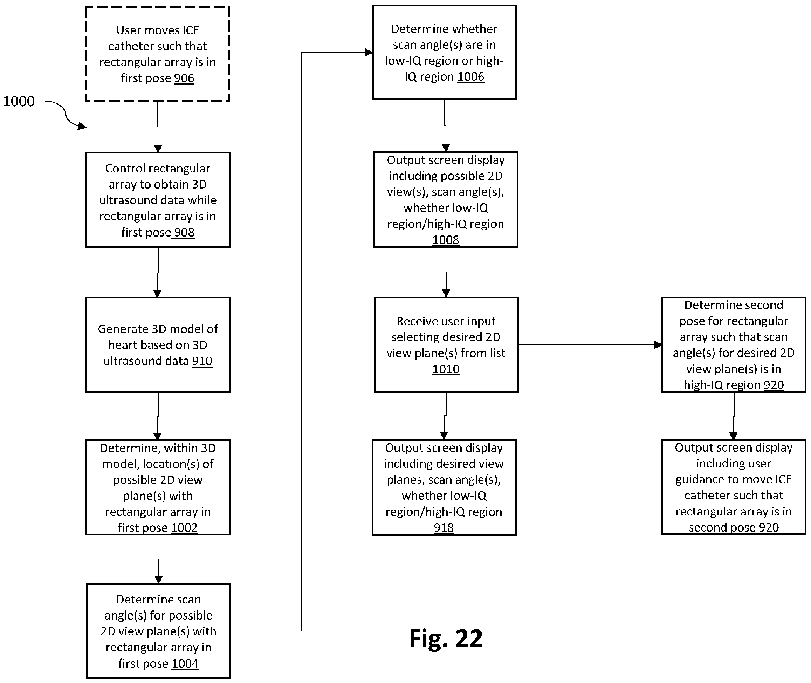

Fig. 22 is a flow chart illustrating a method 1000 similar to the method 900 shown in Fig. 9, according to aspects of the present disclosure. However, in method 1000, the desired 2D view planes are selected after the 3D imaging data has been acquired and model-based segmentation has been performed.

Step 906 of the method 1000 may include the user moving the ICE catheter such that the rectangular array is in a first pose. Step 906 is substantially similar to step 906 in method 900 shown in Fig. 9 and described above.

Step 908 of the method 1000 may include controlling the rectangular array to obtain 3D ultrasound data while the rectangular array is in the first pose. Step 908 is substantially similar to step 908 in method 900 shown in Fig. 9 and described above.

Step 910 of the method 1000 may include generating a 3D model of the heart based on the 3D ultrasound data. Step 910 is substantially similar to step 910 in method 900 shown in Fig. 9 and described above.

Step 1002 of the method 1000 may include determining, within the 3D model, one or more locations of possible 2D view planes with the rectangular array in the first pose. Step 1002 is similar to step 912 in method 900 shown in Fig. 9 and described above. However, unlike in step 912 where the locations of the 2D view planes selected by the user are determined, in step 1002, the user has not selected or indicated any desired 2D view planes. Thus, in step 1002, the locations of one or more possible 2D view planes are determined. The possible 2D view planes may be automatically generated by the system (e.g. control and processing system 130 shown in Fig. 1). In some embodiments, the possible 2D view planes are stored in memory. In some embodiments, the possible 2D view planes are standard view planes of the heart. In some embodiments, the possible 2D view planes are procedure-related or device-related view planes (e.g. 2D view planes of the tricuspid valve).

Step 1004 of the method 1000 may include determining the scan angles (i.e. angular positions) for the possible 2D view planes with the rectangular array in the first pose. Step 1004 is similar to step 914 in method 900 shown in Fig. 9 and described above. However, unlike in step 914 in which scan angles are determined for the selected 2D view planes, in step 1004 the scan angles are determined for the possible 2D view planes.

Step 1006 of the method 1000 may include determining whether the scan angles of the possible 2D view planes are in a low-IQ region or a high-IQ region. Step 1006 is similar to step 916 in method 900 shown in Fig. 9 and described above. However, unlike in step 916 where the scan angles correspond to the selected 2D view planes, in step 1004 the scan angles correspond to the possible 2D view planes.

Step 1008 of the method 1000 may include outputting a screen display including the possible 2D view planes, the scan angles, and whether the possible 2D view planes are in a low-IQ or a high-IQ region.

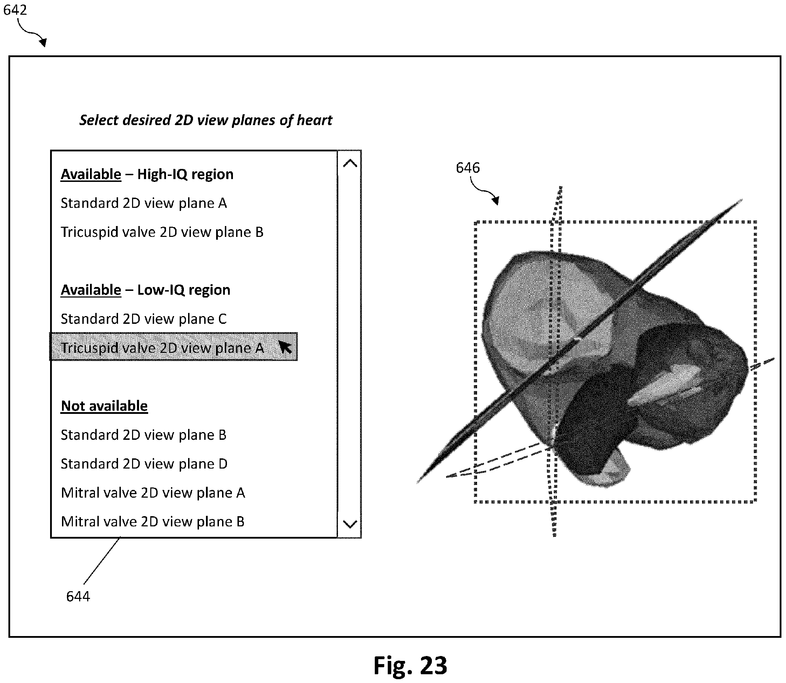

Fig. 23 is an exemplary screen display 642, according to aspects of the present disclosure. In some embodiments, the screen display 642 may include a field 644 including a list of the possible 2D view planes. In the illustrated embodiment, these 2D view planes are grouped by availability and IQ. In the illustrated embodiment, 2D view planes that are available and disposed in a high-IQ region are grouped together, 2D view planes that are available and in a low IQ region are grouped together, and 2D view planes that are unavailable are grouped together. However, the 2D view planes may be listed in any suitable way. For example, in some embodiments, the 2D view planes are listed without dividing them by availability or IQ. In other embodiments, the 2D view planes are divided by type. Each 2D view plane listed is selectable by the user. In some embodiments, the user may select one or more 2D view planes.

In some embodiments, the screen display 642 may also include a field 646 including the adapted 3D model generated from a generic 3D mesh model and the obtained 3D imaging data in step 910. In other embodiments, field 646 may instead include a 3D image associated with the 3D imaging data obtained in step 908. In some embodiments, representations of the possible 2D view planes (e.g. angled 2D plane) relative to the adapted 3D model may be included in field 646. For example, the representations of the 2D view planes may be shown as angled boxes bounded by dotted lines. In some embodiments, the 2D view planes selected in the list in field 644 may be shown as solid in field 646. In some embodiments, the representation is overlaid or superimposed over the 3D model so that the user can easily and effectively understand the location of the possible and/or selected 2D view planes.

Returning to Fig. 22, step 1010 of the method 1000 may include receiving a user input selecting one or more desired 2D view planes from the list of possible 2D view planes. The user may select the desired 2D view planes using any suitable input device. In some embodiments, the input device may be a keyboard, mouse, touchscreen, joystick, button, or any other suitable device. In some embodiments, the user may select a separate button on the screen display directing the system to move to the next step.

Step 918 of the method 1000 may include outputting a second or updated screen display including, for each desired/selected 2D view plane, the selected/desired 2D view plane, the scan angle, and whether the 2D view plane is in a low-IQ region or a high-IQ region. Step 908 is substantially similar to step 908 in method 900 shown in Fig. 9 and described above.

Step 920 of the method 1000 may include determining a second pose for the rectangular array such that the scan angles for the selected/desired 2D view planes are in the high-IQ regions. Step 920 is substantially similar to step 920 in method 900 shown in Fig. 9 and described above.

Step 922 of the method 1000 may include outputting a third or further updated screen display including the user guidance to move the ICE catheter such that the rectangular array is in a second pose. Step 922 is substantially similar to step 922 in method 900 shown in Fig. 9 and described above.

In some embodiments, steps 920-922 may not be performed if all of the selected/desired 2D view planes are in high-IQ regions. In some embodiments, step 918 may not be performed if one or more of the selected/desired view planes are in low-IQ regions.

Further, in some embodiments, the IQ regions of the rectangular array may not only be divided into low-IQ and high-IQ regions (as shown in Figs. 7-8B). Instead, any suitable number of IQ regions may be defined or used.

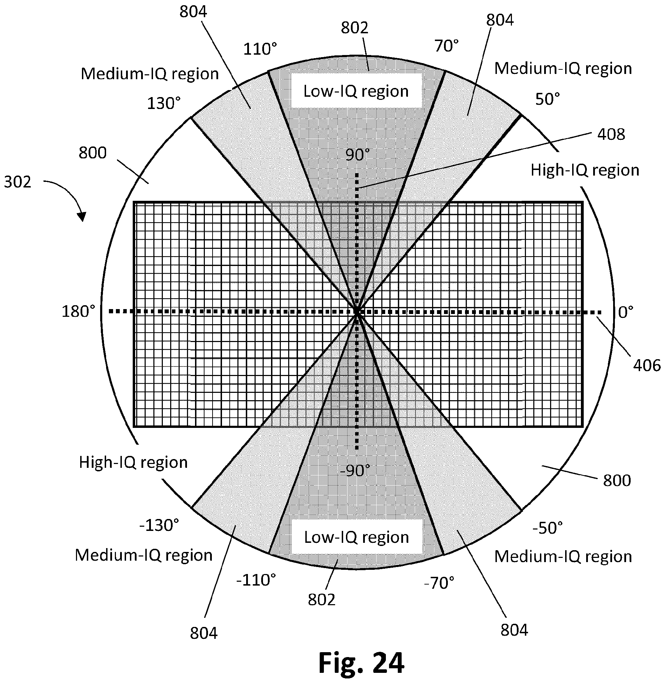

Fig. 24 is a schematic diagram illustrating high-IQ regions 800, low-IQ regions 802, and medium-IQ regions of the rectangular array of imaging elements 302, according to aspects of the present disclosure. Thus, in the illustrated embodiment, there are three different IQ regions defined for the rectangular array 302: low-IQ regions 802, high-IQ regions 800, and medium-IQ regions 804.

The low-IQ regions 802 may be defined about the short axis 408 in which the IQ of images captured along view planes in these regions is low. For example, a low-IQ region 802 may extend from 70 degrees to 110 degrees such that the low-IQ region 414 includes the short axis 408 at 90 degrees. Additionally, another low-IQ region 802 may extend from -110 degrees to -70 degrees such that the low-IQ region 414 includes the short axis 408 at -90 degrees.

In some embodiments, high-IQ regions may be defined about the long axis 406 in which the IQ of images captured along view planes in these regions is high. For example, a high-IQ region 800 may extend from -50 degrees to 50 degrees such that the high-IQ region 800 includes the long axis 406 at 0 degrees. Additionally, another high-IQ region 800 may extend from 130 degrees to -130 degrees from the long axis 406 such that the high-IQ region 800 includes the long axis 408 at 180 degrees. These low-IQ 414 and high-IQ 416 regions are exemplary.

Moreover, medium-IQ regions 804 may be defined between the low-IQ 802 and high-IQ 800 regions. The IQ of images captured along view planes within the medium-IQ regions 804 may be lower than that of the high-IQ regions 800, but higher than that of the low-IQ regions 802. Thus, the IQ of images captured along view planes in the medium IQ regions 804 may still be acceptable to a user. For example, medium-IQ regions 804 may extend from 50 degrees to 70 degrees, from 110 degrees to 130 degrees, from -130 degrees to -110 degrees, and from -70 degrees to -50 degrees.

Thus, in step 920 of method 900 shown in Fig. 9, when determining the second pose for the rectangular array, the system may determine a pose in which the selected 2D view planes that were previously disposed in low-IQ regions are disposed in either high-IQ regions or medium-IQ regions. Thus, this may advantageously make it easier to recapture 2D images along the selected 2D view planes by allowing for more ranges of acceptable IQ-regions. In some embodiments, this may make it easier to recapture multiple planes at the same second pose.

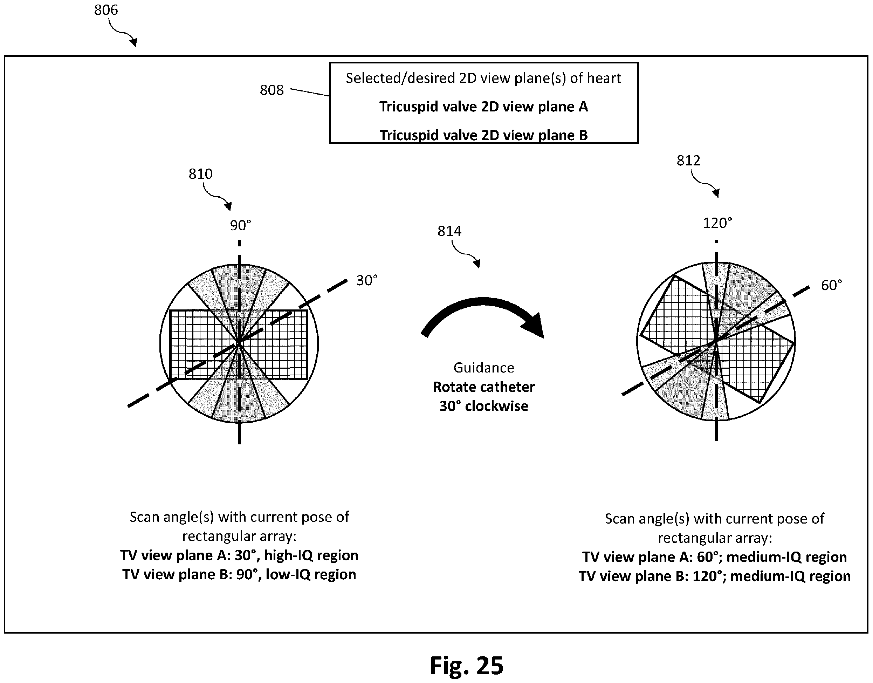

Fig. 25 is an exemplary further updated screen display 806 that may be displayed in step 922 of method 900 (shown in Fig. 9), according to aspects of the present disclosure. The screen display 806 may include various fields. In some embodiments, the screen display 806 may include a field 808 identifying the selected/desired 2D view planes. In the illustrated embodiment, two 2D view planes have been selected by the user: the tricuspid valve 2D view plane A and the tricuspid valve view plane B.

Moreover, in some embodiments, the screen display 806 may include a field 810 that shows the scan angles of the selected 2D view planes at the first pose (i.e. the first scan angles). In the illustrated embodiment, the first scan angle of tricuspid valve (TV) view plane A is 30 degrees, which is within the defined high-IQ region and the first scan angle of TV view plane B is 90 degrees, which is within the defined low-IQ region. The field 810 may include the numerical value of the 2D view planes (e.g. 30 degrees for plane A, 90 degrees for plane B) and the IQ region that the 2D view plane is disposed in (e.g. high-IQ region for TV plane A, low-IQ region for TV plane B). In some embodiments, field 810 also includes a schematic diagram (e.g. graphical representation) of the rectangular array and a representation (e.g. dotted line) of the 2D view planes at the determined scan angles relative to the rectangular array. A representation of the low-IQ, high-IQ, and medium-IQ regions relative to the rectangular array may also be included in field 810. These representations may be overlaid or superimposed over the schematic diagram of the rectangular array so that the user can easily and effectively understand the location of the selected 2D view planes.

The screen display 806 may also include a field 814 that includes the user guidance. The user guidance may include a description of the recommended movement (e.g. text describing the movement). For example, in the illustrated embodiment, the recommended movement is to rotate the rectangular array. Moreover, the user guidance may also include a numerical value representing the extent of the recommended movement and/or a direction of the recommended movement. For example, in the illustrated embodiment, the user guidance recommends that the user rotate the rectangular array 30 degrees clockwise. The user guidance may further include a visual representation (e.g. arrow) associated with the recommended movement.

The screen display 806 may also include a field 812 that shows the scan angles of the selected 2D view planes at the second pose (i.e. the second scan angles). In the illustrated embodiment, the second scan angle of TV plane A is 120 degrees and the second scan angle of TV plane B is 60 degrees. Therefore, both of the second scan angles are within the medium-IQ region. This may advantageously prevent the desired 2D view planes being in an unacceptable low-IQ region by choosing a compromise position in which both of the desired 2D view planes are in an acceptable IQ region (e.g. a medium-IQ region).

The field 812 may include the numerical value of the 2D view planes (e.g. 120 degrees for TV plane A, 60 degrees for TV plane B) and the IQ regions that the 2D view planes are disposed in (e.g. high-IQ region for TV plane A, low-IQ region for TV plane B). In some embodiments, field 812 also includes a schematic diagram (e.g. graphical representation) of the rectangular array and representations (e.g. dotted line) of the 2D view planes at the determined scan angles relative to the rectangular array. A representation of the low-IQ, high-IQ, and medium-IQ regions relative to the rectangular array may also be included in field 812. These representations may be overlaid or superimposed over the schematic diagram of the rectangular array so that the user can easily and effectively understand the location of the selected 2D view planes. The schematic diagram of the rectangular array may be rotated relative to that shown in field 810 in accordance with the user guidance.

Further, in some embodiments, the rectangular array may not be divided into IQ regions at all and, instead, each angular position around the rectangular array may have a defined quality score. In other words, there may be a continuous range of values of the IQ around the matrix of the rectangular array.

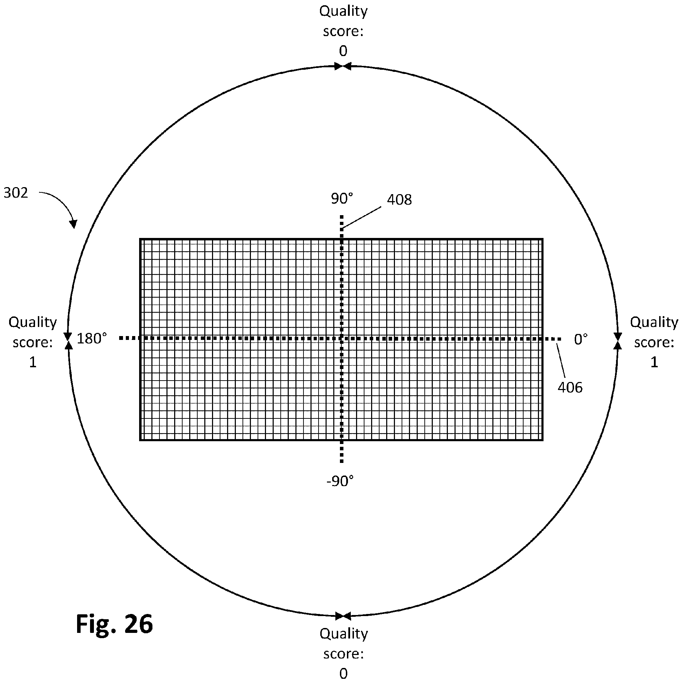

Fig. 26 is a schematic diagram illustrating exemplary quality scores for the rectangular array of imaging elements 302, according to aspects of the present disclosure. For example, a quality score of 1 may be defined for view planes along the long axis 406 at 0 degrees and 180 degrees and a quality score of 0 may be defined for view planes along the short axis 408 at 90 degrees and -90 degrees. Thus, a quality score of 1 may be the highest quality score (because the IQ of images corresponding to view planes along the long axis 406 is the highest). Accordingly, a quality score of 0 may be the lowest quality score (because the IQ of images corresponding to view planes along the short axis 408 is the lowest). Thus, angles between the long axis 406 and short axis 408 may be given a quality score of between 0 and 1 depending on the angular distance from the short axis 408 / long axis 406. For example, a quality score of 0.5 is defined at the angular position of 45 degrees, 135 degrees, -135 degrees, and -45 degrees.

Thus, when a quality score is used instead of IQ regions, method 900 (shown in Fig. 9) may be modified slightly. In step 916 of method 900, instead of determining whether the scan angles are in a low-IQ or high-IQ region, the quality scores of each scan angle is determined based on the distance from the long/short axis. In step 918, instead of outputting whether the selected 2D view plane is in a low-IQ or high-IQ region, the quality score is output. In some embodiments, the screen display may include an indication of how high or low the quality score is (e.g. through color coding or a descriptive indicator). In step 920, instead of determining a second pose in which the selected 2D view planes are in a high-IQ region, the second pose is determined by maximizing the quality score of the one or more selected 2D view planes while minimizing the movement of the rectangular array required.

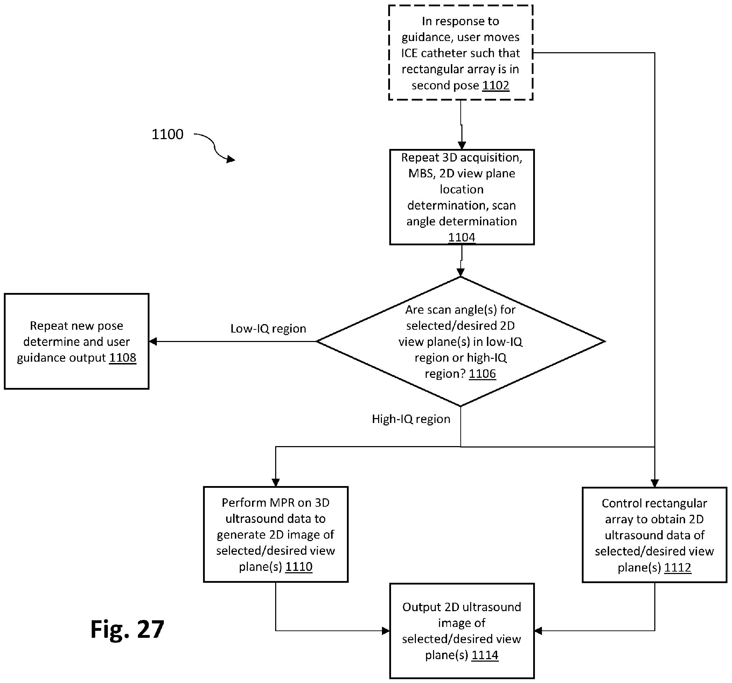

Fig. 27 is a flow chart illustrating a method 1100 for obtaining a 2D image of the selected 2D view planes at the second pose, according to aspects of the present disclosure. Thus, the method 1100 may be performed after step 922 in either method 900 shown in Fig. 9 or method 1000 shown in Fig. 22.

Step 1102 of the method 1100 may include, in response to the outputted user guidance, the user moving the ICE catheter such that the rectangular array is in the second pose. In some embodiments, the movement may include flexing the rectangular array left/right or anterior/posterior, advancing or retracting the ICE catheter that includes the rectangular array into or out of the body, rotating the handle of the ICE catheter towards or away from the user (i.e. clock or counterclock).

Step 1104 of the method 1100 may include repeating 3D acquisition, model-based segmentation (MBS), 2D view plane location determination, and scan angle determination (steps 908-914). Steps 908-914 are described above in reference to Fig. 9.

Step 1106 of the method 1100 may include determining whether the scan angles for the selected 2D view planes are in low-IQ regions or high-IQ regions in the second pose. Step 1106 is substantially similar to step 916, which is described above in reference to Fig. 9.

If the scan angles of one or more 2D view planes are in a low-IQ region, the method 1100 proceeds to step 1108. Step 1108 may include repeating new pose determination and user guidance output (steps 920-922). Steps 920-922 are described above in reference to Fig. 9.

If the scan angles of all of the one or more 2D view planes are in high-IQ regions, then the method 1100 proceeds to step 1110 or step 1112.

Step 1110 may include performing MPR on the 3D imaging data (i.e. 3D ultrasound data) at the second pose to generate 2D images of the one or more selected/desired view planes. In some embodiments, the scan angle of the selected 2D view planes may be used to determine the location of the desired 2D image in the 3D image.

In step 1112 of the method 1100 may include controlling the rectangular array to obtain 2D imaging data (e.g. 2D ultrasound data) of the one or more selected/desired 2D view planes. Thus, instead of using the previously acquired 3D imaging data at the second pose (as in step 1110), new 2D imaging data may be obtained. Although, in some embodiments, the rectangular array may capture 3D imaging data in this step, because the scan angles of the desired 2D view planes are known, the rectangular array may be controlled to capture 2D imaging data at those known scan angles.

In some embodiments, after step 1102, instead of performing step 1104 and acquiring 3D imaging data, step 1112 is performed to acquire 2D imaging data at the selected imaging plane. Thus, the process of confirming that the desired 2D view planes are in high-IQ regions in the second pose may be skipped or bypassed.

Step 1114 of the method 1100 may include outputting one or more 2D images (e.g. 2D ultrasound images) of the selected/desired 2D view planes. In some embodiments, a single 2D image may be output to a screen display. In embodiments in which more than one 2D view planes were selected, the screen display may allow the user to toggle between the 2D images of the selected 2D view planes. In other embodiments, all of the 2D images may be output to the screen display simultaneously.

Further, in some embodiments, it may be desirable to obtain 2D ultrasound images simultaneously with the 3D imaging data so that the 2D ultrasound images are output to a display while the system performs further analysis of the 3D imaging data to improve the IQ of the desired 2D view planes.

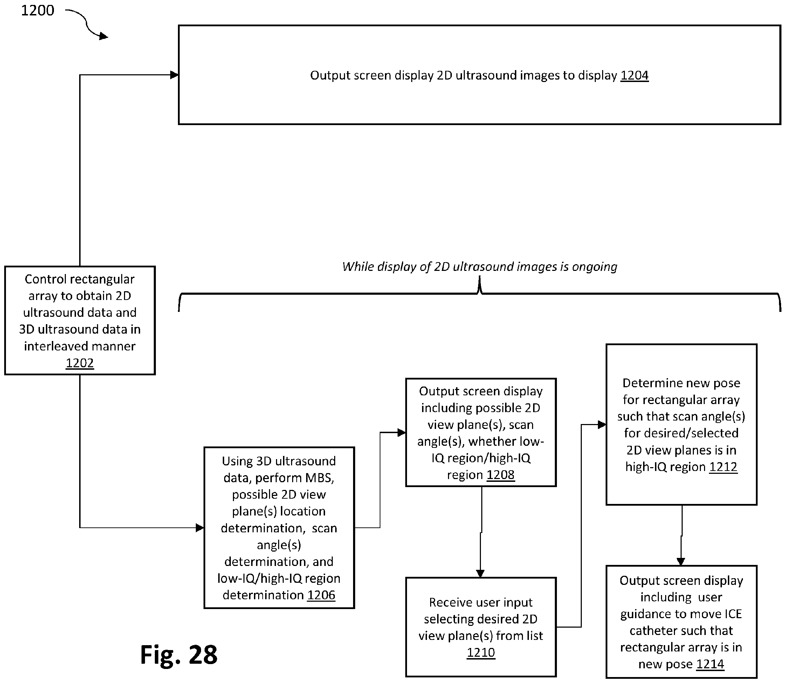

Fig. 28 is a flow chart illustrating a method 1200 in which 2D ultrasound images are output to a display while further analysis of the 3D imaging data is performed, according to aspects of the present disclosure.

Step 1202 of the method 1200 may include controlling the rectangular array to obtain 2D ultrasound data and 3D ultrasound imaging data at the same time in an interleaved manner. Thus, the 2D ultrasound data may be collected along with the 3D ultrasound imaging data.

Step 1206 of the method 1200 may include, using the 3D ultrasound data, performing model-based segmentation (MBS), location determination for one or more possible 2D view planes, scan angle determination for the one or more possible 2D view planes, and determination of whether the one or more scan angles of the one or more possible 2D view planes are within a low-IQ region or a high-IQ region. Step 1206 is substantially similar to steps 910, 1002, 1004, and 1006 of method 1000 shown in Fig. 22 and described above.

Step 1208 of the method 1200 may include outputting a screen display including the one or more possible 2D view planes, the scan angles, and whether the possible 2D view planes are in a low-IQ or a high-IQ region. Step 1208 is substantially similar to step 1008 of method 1000 shown in Fig. 22 and described above.

Step 1210 of the method 1200 may include receiving a user input selecting the desired 2D view planes from the list of possible 2D view planes. Step 1210 is substantially similar to step 1010 of method 1000 shown in Fig. 22 and described above.

Step 1212 of the method 1200 may include determining a new pose for the rectangular array such that one or more of the scan angles of the selected/desired 2D view planes are in the high-IQ regions. Step 1212 is substantially similar to step 920 of methods 900 and 1000 shown in Figs. 9 and 22 and described above.

Step 1214 of the method 1200 may include outputting a screen display including the user guidance to move the ICE catheter such that the rectangular array is in the new pose. Step 1212 is substantially similar to step 922 of methods 900 and 1000 shown in Figs. 9 and 22 and described above.

Step 1204 of the method 1200 may include outputting a screen display including 2D ultrasound images to a display. Step 1204 may be performed while steps 1206-1214 are being performed (i.e. while steps 1206-1214 are ongoing). In some embodiments, the screen display in step 1204 may be output to the same display as the screen displays in steps 1208 and/or 1214. In other embodiments, the screen display in step 1204 may be output to a different display than the screen displays in steps 1208 and 1214.

Fig. 29 is a schematic diagram of a processor circuit 1600, according to aspects of the present disclosure. The processor circuit 1600 may be implemented in the control and processing system 130, the system 100, or other devices or workstations (e.g., third-party workstations, network routers, etc.), or on a cloud processor or other remote processing unit, as necessary to implement the method. As shown, the processor circuit 1600 may include a processor 1610, a memory 1612, and a communication module 1614. These elements may be in direct or indirect communication with each other, for example via one or more buses.

The processor 1610 may include a central processing unit (CPU), a digital signal processor (DSP), an ASIC, a controller, or any combination of general-purpose computing devices, reduced instruction set computing (RISC) devices, application-specific integrated circuits (ASICs), field programmable gate arrays (FPGAs), or other related logic devices, including mechanical and quantum computers. The processor 1610 may also comprise another hardware device, a firmware device, or any combination thereof configured to perform the operations described herein. The processor 1610 may also be implemented as a combination of computing devices, e.g., a combination of a DSP and a microprocessor, a plurality of microprocessors, one or more microprocessors in conjunction with a DSP core, or any other such configuration.

The memory 1612 may include a cache memory (e.g., a cache memory of the processor 1610), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an aspect, the memory 1612 includes a non-transitory computer-readable medium. The memory 1612 may store instructions 1616. The instructions 1616 may include instructions that, when executed by the processor 1610, cause the processor 1610 to perform the operations described herein. Instructions 1616 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 1614 can include any electronic circuitry and/or logic circuitry to facilitate direct or indirect communication of data between the processor circuit 1600, and other processors or devices. In that regard, the communication module 1614 can be an input/output (I/O) device. In some instances, the communication module 1614 facilitates direct or indirect communication between various elements of the processor circuit 1600 and/or the system 800. The communication module 1614 may communicate within the processor circuit 1600 through numerous methods or protocols. Serial communication protocols may include but are not limited to United States Serial Protocol Interface (US SPI), Inter-Integrated Circuit (I²C), Recommended Standard 232 (RS-232), RS-485, Controller Area Network (CAN), Ethernet, Aeronautical Radio, Incorporated 429 (ARINC 429), MODBUS, Military Standard 1553 (MIL-STD-1553), or any other suitable method or protocol. Parallel protocols include but are not limited to Industry Standard Architecture (ISA), Advanced Technology Attachment (ATA), Small Computer System Interface (SCSI), Peripheral Component Interconnect (PCI), Institute of Electrical and Electronics Engineers 488 (IEEE-488), IEEE-1284, and other suitable protocols. Where appropriate, serial and parallel communications may be bridged by a Universal Asynchronous Receiver Transmitter (UART), Universal Synchronous Receiver Transmitter (USART), or other appropriate subsystem.

External communication (including but not limited to software updates, firmware updates, preset sharing between the processor and central server, or readings from the leaflet puncture and slitting device) may be accomplished using any suitable wireless or wired communication technology, such as a cable interface such as a universal serial bus (USB), micro USB, Lightning, or FireWire interface, Bluetooth, Wi-Fi, ZigBee, Li-Fi, or cellular data connections such as 2G/GSM (global system for mobiles) , 3G/UMTS (universal mobile telecommunications system), 4G, long term evolution (LTE), WiMax, or 5G. For example, a Bluetooth Low Energy (BLE) radio can be used to establish connectivity with a cloud service, for transmission of data, and for receipt of software patches. The controller may be configured to communicate with a remote server, or a local device such as a laptop, tablet, or handheld device, or may include a display capable of showing status variables and other information. Information may also be transferred on physical media such as a USB flash drive or memory stick.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. An apparatus, comprising:
a rectangular ultrasound array (302) comprising a long axis (406) and a short axis (408) perpendicular to the long axis;
a processor (130) configured for communication with the rectangular ultrasound array, wherein the processor is configured to:
control the rectangular ultrasound array to obtain three-dimensional (3D) ultrasound data of an anatomy of a patient while the rectangular ultrasound array is in a first pose;
determine a location of a two-dimensional (2D) view plane of the anatomy, within a 3D model of the anatomy based on at least the 3D ultrasound data;
determine, for the 2D view plane, a first scan angle associated with the first pose of the rectangular ultrasound array, wherein the first scan angle comprises an orientation of the 2D view plane relative to at least one of the long axis or the short axis;
determine, for the first scan angle, a first image quality associated with the orientation of the 2D view plane relative to at least one of the long axis or the short axis; and
output, to a display (132) in communication with the processor, a screen display comprising at least one of the location of the 2D view plane, the first scan angle, or the first imaging quality.

2. The apparatus of claim 1, wherein the screen display comprises:
a graphical representation (634) of the rectangular ultrasound array in the first pose;
a plurality of image quality regions overlaid on the graphical representation; and
an indicator identifying the first scan angle relative to a plurality of image quality regions.

3. The apparatus of claim 1,
wherein the processor is configured to determine, for the 2D view plane, a second scan angle comprising a second image quality that is relatively higher than the first image quality,
wherein the screen display comprises at least one of the second scan angle or the second image quality.

4. The apparatus of claim 3,
wherein the processor is configured to generate user guidance (638) to move the rectangular ultrasound array to a second pose,
wherein, in the second pose, the 2D view plane comprises the second scan angle, and
wherein the screen display further comprises the user guidance.

5. The apparatus of claim 4, wherein, after movement of the rectangular ultrasound array to the second pose, the processor is further configured to control the rectangular ultrasound array to obtain further ultrasound data of the anatomy.

6. The apparatus of claim 4, wherein the screen display further comprises:
at least one of a text or a visual representation of the user guidance (638);
a graphical representation (636) of the rectangular ultrasound array in the second pose;
a plurality of image quality regions overlaid on the graphical representation; and
an indicator identifying the second scan angle relative to plurality of image quality regions.

7. The apparatus of claim 4, wherein, to generate the user guidance, the processor is configured to perform a subtraction using a value of the first scan angle and a value of the second scan angle.

8. The apparatus of claim 1, wherein, before the determination of the location of the 2D view plane, the processor is configured to receive a user input identifying the 2D view plane.

9. The apparatus of claim 8,
wherein, before the determination of the location of the 2D view plane, the processor is configured to output a list (602) of a plurality of 2D view planes, and
wherein the user input comprises selection of the 2D view plane from the list.

10. The apparatus of claim 1,
wherein the processor is configured to determine a location for each of a plurality of 2D view planes within the 3D model, and
wherein the 2D view plane is one of the plurality of 2D view planes.

11. The apparatus of claim 10, wherein processor is configured to:
determine a plurality of first scan angles for the plurality of 2D view planes;
determine a plurality of first image qualities for the plurality of first scan angles;
output, to the display, a list (602) based on at least one of the location for each of the plurality of 2D view planes, the plurality of first scan angles, or the plurality of first image qualities; and
receive a user input selecting the 2D view plane from the list.

12. The apparatus of claim 1,
wherein, to determine the first image quality, the processor is configured to determine that the first scan angle extends within one of a plurality of image quality regions,
wherein the plurality of image quality regions comprises:
a first image quality region comprising a first scan angle range proximate to the long axis of the rectangular ultrasound array; and
a second image quality region comprising a second scan angle range proximate to the short axis of the rectangular ultrasound array.

13. The apparatus of claim 12,
wherein the plurality of image quality regions further comprises a third image quality region comprising a third scan angle range between the first scan angle range and the second scan angle range.

14. The apparatus of claim 13, wherein the processor is configured to:
determine an additional location of an additional 2D view plane of the anatomy, within the 3D model of the anatomy based on the 3D ultrasound data,
determine, for the additional 2D view plane, an additional first scan angle associated with the first pose of the rectangular ultrasound array, wherein the additional first scan angle comprises an orientation of the additional 2D view plane relative to at least one of the long axis or the short axis,
determine, for the additional first scan angle, an additional first image quality associated with the orientation of the 2D view plane relative to at least one of the long axis or the short axis;
determine, for the 2D view plane and the additional 2D view plane, a second scan angle and an additional second scan angle, wherein the second scan angle and the additional second scan angle are within either the first image quality region or the third image quality region; and
generate user guidance to move the rectangular ultrasound array to a second pose in which the 2D view plane comprises the second scan angle and the additional 2D view plane comprises the additional second scan angle, wherein the screen display further comprises the user guidance.

15. A method comprising the steps of:
- providing an apparatus (100), comprising a rectangular ultrasound array (302) comprising a long axis (406) and a short axis (408) perpendicular to the long axis;
- obtaining (908) three-dimensional (3D) ultrasound data of an anatomy of a patient while the rectangular ultrasound array is in a first pose ;
- determining (912) a location of a two-dimensional (2D) view plane of the anatomy, within a 3D model of the anatomy based on at least the 3D ultrasound data;
- determining (914), for the 2D view plane, a first scan angle associated with the first pose of the rectangular ultrasound array, wherein the first scan angle comprises an orientation of the 2D view plane relative to at least one of the long axis or the short axis;
- determining (916), for the first scan angle, a first image quality associated with the orientation of the 2D view plane relative to at least one of the long axis or the short axis; and
- displaying a screen (918) comprising at least one of the location of the 2D view plane, the first scan angle, or the first imaging quality.
